# EUROPEAN PATENT APPLICATION

(11) **EP 4 595 871 A1**
(43) Date of publication of application: **06.08.2025**
(21) Application number: 23872160.9
(22) Date of filing: 22.09.2023
(51) Int. Cl.: A61B 3/103, A61B 3/11

(54) **OPHTHALMIC DEVICE, METHOD FOR CONTROLLING OPHTHALMIC DEVICE, AND PROGRAM**

(30) Priority: 27.09.2022 JP 2022153742
(71) Applicant: TOPCON CORPORATION, Tokyo 174-8580 (JP)
(72) Inventor: TATARA, Yoko, Tokyo 174-8580 (JP); FURUGAICHI, Taketo, Tokyo 174-8580 (JP)
(74) Representative: Gagel, Roland
(86) International application number: PCT/JP2023/034449
(87) International publication number: WO 2024/070934

(57) **Abstract**

This ophthalmic device includes a measurement optical system, an acquisition unit, a control unit, and a calculation unit. The measurement optical system includes a focusing lens. The measurement optical system measures the wavefront aberration of an eye under examination in which an intraocular lens is to be placed, and acquires a Hartmann image. The acquisition unit acquires at least intraocular lens information representing the optical characteristics of the intraocular lens. The control unit moves the focusing lens to a position corresponding to the focal length of the intraocular lens, the focal length being determined on the basis of the intraocular lens information, and causes a Hartmann image to be acquired by the measurement optical system. The calculation unit calculates, on the basis of the Hartmann image, the refractive power of the eye under examination, using a computation process method corresponding to the intraocular lens information.

## Description

### [TECHNICAL FIELD]

This invention relates to ophthalmic apparatus, method of controlling the ophthalmic apparatus, and program.

### [BACKGROUND ART]

When a cataract progresses, it is common for cataract surgery to be performed. In the cataract surgery, a content of a lens capsule is removed and an intraocular lens (IOL, hereinafter) is implanted in the lens capsule. There are various types of IOLs. The examinee needs to select an appropriate type of IOL, taking into the contrast of image, the brightness, the distant visual acuity, the near visual acuity, etc. After the surgery, the dioptric power of the eye to be examined wearing the IOL is measured, and the view and/or the recovery of visual acuity vision and other factors are checked.

There have been several proposals for ophthalmic apparatuses for examining the eye to be examined wearing such an IOL. For example, Patent Document 1 discloses a method of acquiring a transillumination image of the eye to be examined and of determining from the acquired transillumination image whether or not the IOL is worn by the eye to be examined. For example, Patent Document 2 discloses a method of obtaining a dioptric power using a point image group of a part of point images obtained using wavefront aberration information. For example, Patent Document 3 discloses a method of measuring a dioptric power of an eye to be examined wearing the IOL by projecting a ring pattern.

### [PRIOR ART DOCUMENTS]

### [PATENT DOCUMENTS]

[Patent Document 1] Japanese Unexamined Patent Publication No. 2014-209994
[Patent Document 2] Japanese Unexamined Patent Publication No. 2017-213124
[Patent Document 3] Japanese Unexamined Patent Publication No. 2021-083940

### [SUMMARY OF THE INVENTION]

### [PROBLEMS TO BE SOLVED BY THE INVENTION]

In the conventional methods, the dioptric power is calculated using a uniform method regardless of the type of IOL. Therefore, there is a problem that the reliability of the calculation result of the dioptric power decreases depending on the type of IOL implanted in the eye to be examined.

The present invention has been made in view of such circumstances, and one of objects of the present invention is to provide a new technique for improving reliability of calculation result of a dioptric power of an eye to be examined wearing an IOL.

### [MEANS OF SOLVING THE PROBLEMS]

One aspect of embodiments is an ophthalmic apparatus including: a measurement optical system including a focusing lens, and configured to measure wavefront aberration of an eye to be examined wearing an intraocular lens to acquire a Hartmann image; an acquisition unit configured to acquire intraocular lens information representing at least an optical characteristics of the intraocular lens; a controller configured to move the focusing lens to a position corresponding to a focal point distance of the intraocular lens determined based on the intraocular lens information and to cause the measurement optical system to acquire the Hartmann image; and a calculator configured to calculate a dioptric power of the eye to be examined based on the Hartmann image using an arithmetic processing method corresponding to the intraocular lens information.

Another aspect of the embodiments is a method of controlling an ophthalmic apparatus including a measurement optical system including a focusing lens and configured to measure wavefront aberration of an eye to be examined wearing an intraocular lens to acquire a Hartmann image. The method of controlling the ophthalmic apparatus includes: an acquisition step of acquiring intraocular lens information representing at least an optical characteristics of the intraocular lens; a control step of moving the focusing lens to a position corresponding to a focal point distance of the intraocular lens determined based on the intraocular lens information and of causing the measurement optical system to acquire the Hartmann image; and a calculation step of calculating a dioptric power of the eye to be examined based on the Hartmann image using an arithmetic processing method corresponding to the intraocular lens information.

Still another aspect of the embodiments is a program of causing a computer to execute each step of method of controlling the ophthalmic apparatus described above.

### [EFFECTS OF THE INVENTION]

According to the present invention, a new technique for improving reliability of calculation result of a dioptric power of an eye to be examined wearing an IOL can be provided.

### [BRIEF EXPLANATION OF THE DRAWINGS]

[FIG. 1] FIG. 1 is a schematic diagram illustrating an example of a configuration of an optical system of an ophthalmic apparatus according to embodiments.
[FIG. 2] FIG. 2 is a schematic diagram illustrating an example of a configuration of an optical system of the ophthalmic apparatus according to the embodiments.
[FIG. 3] FIG. 3 is a schematic diagram illustrating an example of a configuration of an optical system of the ophthalmic apparatus according to the embodiments.
[FIG. 4] FIG. 4 is a schematic diagram illustrating an example of a configuration of an optical system of the ophthalmic apparatus according to the embodiments.
[FIG. 5] FIG. 5 is a diagram for explaining an operation of the ophthalmic apparatus according to the embodiments.
[FIG. 6] FIG. 6 is a schematic diagram illustrating an example of a configuration of a processing system of the ophthalmic apparatus according to the embodiments.
[FIG. 7] FIG. 7 is a schematic diagram illustrating an example of a configuration of a processing system of the ophthalmic apparatus according to the embodiments.
[FIG. 8] FIG. 8 is a schematic diagram illustrating an example of a configuration of a processing system of the ophthalmic apparatus according to the embodiments.
[FIG. 9] FIG. 9 is a schematic diagram illustrating an example of a configuration of a processing system of the ophthalmic apparatus according to the embodiments.
[FIG. 10] FIG. 10 is a schematic diagram for explaining a multifocal refractive type IOL according to the embodiments.
[FIG. 11] FIG. 11 is a schematic diagram for explaining the multifocal refractive type IOL according to the embodiments.
[FIG. 12] FIG. 12 is a schematic diagram illustrating an example of a configuration of a processing system of the ophthalmic apparatus according to the embodiments.
[FIG. 13] FIG. 13 is a schematic diagram for explaining a multifocal diffractive type IOL according to the embodiments.
[FIG. 14] FIG. 14 is a schematic diagram illustrating an example of a configuration of a processing system of the ophthalmic apparatus according to the embodiments.
[FIG. 15] FIG. 15 is a schematic diagram for explaining an extended depth of focus type IOL according to the embodiments.
[FIG. 16] FIG. 16 is a flowchart of an example of an operation of the ophthalmic apparatus according to the embodiments.
[FIG. 17] FIG. 17 is a flowchart of an example of an operation of the ophthalmic apparatus according to the embodiments.
[FIG. 18] FIG. 18 is a flowchart of an example of an operation of the ophthalmic apparatus according to the embodiments.
[FIG. 19] FIG. 19 is a flowchart of an example of an operation of the ophthalmic apparatus according to the embodiments.
[FIG. 20] FIG. 20 is a flowchart of an example of an operation of the ophthalmic apparatus according to the embodiments.
[FIG. 21] FIG. 21 is a flowchart of an example of an operation of the ophthalmic apparatus according to the embodiments.
[FIG. 22] FIG. 22 is a flowchart of an example of an operation of the ophthalmic apparatus according to the embodiments.
[FIG. 23] FIG. 23 is a flowchart of an example of an operation of the ophthalmic apparatus according to the embodiments.
[FIG. 24] FIG. 24 is a flowchart of an example of an operation of the ophthalmic apparatus according to the embodiments.
[FIG. 25] FIG. 25 is a flowchart of an example of an operation of the ophthalmic apparatus according to the embodiments.
[FIG. 26] FIG. 26 is a flowchart of an example of an operation of the ophthalmic apparatus according to the embodiments.
[FIG. 27] FIG. 27 is a flowchart of an example of an operation of the ophthalmic apparatus according to the embodiments.
[FIG. 28] FIG. 28 is a flowchart of an example of an operation of the ophthalmic apparatus according to the embodiments.
[FIG. 29] FIG. 29 is a flowchart of an example of an operation of an ophthalmic apparatus according to a first modification example of the embodiments.
[FIG. 30] FIG. 30 is a flowchart of an example of an operation of an ophthalmic apparatus according to a second modification example of the embodiments.

### [MODES FOR CARRYING OUT THE INVENTION]

Referring now to the drawings, exemplary embodiments of an ophthalmic apparatus, a method of controlling the ophthalmic apparatus, and a program according to the present invention are described below. Any of the contents of the documents cited in the present specification and arbitrary known techniques may be applied to the embodiments below.

An ophthalmic apparatus according to embodiments includes a measurement optical system including a focusing lens, and configured to measure wavefront aberration of an eye to be examined wearing an IOL (intraocular lens) to acquire a Hartmann image. For example, by implanting the IOL in a lens capsule whose contents have been surgically removed, the eye to be examined can wear the IOL. The ophthalmic apparatus moves the focusing lens to a position corresponding to a focal point distance of the IOL, the focal point distance being determined based on IOL information representing at least optical characteristics of the IOL, and calculates a dioptric power of the eye to be examined based on the Hartmann image acquired by the measurement optical system, using an arithmetic processing method corresponding to the IOL information. In some embodiments, the ophthalmic apparatus calculates the dioptric power based on the Hartmann image within a region demarcated based on pupil diameter information representing a pupil diameter.

The IOL information includes, for example, at least one of information representing the number of focal points (the number of focal point distances) of the IOL, information representing positions of areas with different focal point distances in the IOL, information representing whether or not to utilize the refractive phenomenon of light, information representing whether or not to utilize the diffraction phenomenon of light, or information representing whether or not to have a deep depth of focus. Such IOL information can be identified by a predetermined type of IOL. Examples of the type of IOL include a monofocal type and a multifocal type. Examples of the multifocal type include a multifocal diffractive type and a multifocal refractive type. In some embodiments, the multifocal type includes an Extended of Depth of Focus (hereinafter referred to as EDoF) type. Such IOL information is obtained by designated by a user (examinee, examiner, doctor, etc.) using an operation unit or by analyzing an anterior segment image or a transillumination image of the eye to be examined wearing the IOL to determine the type of IOL.

The ophthalmic apparatus changes the method of driving the focusing lens and the method of calculating the dioptric power according to the IOL information, and calculates the dioptric power according to the type of IOL. The wavefront aberration information includes a Hartmann image obtained in a state of being focused on the eye to be examined in accordance with the focal point distance (or average focal point distance) of the IOL, or a plurality of Hartmann images, each of which is obtained in states of being focused on the eye to be examined in accordance with each of a plurality of focal point distances of the IOL. The dioptric power includes, for example, a spherical power (S), a cylindrical power (C), and an astigmatic axis angle (A). In some embodiments, the ophthalmic apparatus calculates the dioptric power for each focal point distance of the IOL (according to focal point distance of the IOL).

Further, according to the type of IOL, at least one of a plurality of point images that make up the acquired Hartmann image is separated into two or more separated point images. Each of the two or more separated point images corresponds to the focal point distance of the IOL. Therefore, the ophthalmic apparatus can classify the two or more separated point images, each of which corresponds to each point image, into any one of the two or more point image groups based on the IOL information, and can calculate the dioptric power using a known method in that a Zernike polynomial approximation is performed for each point image group.

In some embodiments, the ophthalmic apparatus acquires pupil diameter information representing a pupil diameter of the eye to be examined, and calculates the dioptric power based on the wavefront aberration information within a region demarcated based on the acquired pupil diameter information. For example, the ophthalmic apparatus normalizes the wavefront aberration information using the pupil diameter information, and calculates the dioptric power using a known method in that a Zernike polynomial approximation is performed using the normalized wavefront aberration information.

Thereby, the dioptric power can be obtained by changing a measurement method in accordance with the type worn by the eye to be examined. As a result, the reliability of the calculation result of the dioptric power of the eye to be examined wearing an IOL can be improved.

The ophthalmic apparatus according to the embodiments realizes the function(s) of the ophthalmic information processing apparatus according to the embodiments. A method of controlling the ophthalmic apparatus or ophthalmic information processing method according to the embodiments includes one or more steps for realizing the processing executed by a processor (computer) in the ophthalmic apparatus or ophthalmic information processing apparatus according to the embodiments. A program according to the embodiments causes the processor to execute each step of the method of controlling the ophthalmic apparatus or the ophthalmic information processing method according to the embodiments. A recording medium (storage medium) according to the embodiments is a computer readable non-transitory recording medium (storage medium) on which the program according to the embodiments is recorded.

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program.

Hereinafter, the ophthalmic apparatus according to the embodiments will be described as having the functions of the ophthalmic information processing apparatus according to the embodiments. Further, the types of the IOL are assumed to include the monofocal type, the multifocal type, and the EDoF type. In this case, unless otherwise mentioned, a case where the number of focal points of the multifocal type IOL is "2" will be described. However, the configuration according to the embodiments can be applied to a case where the number of focal points is three or more.

The ophthalmic apparatus according to the embodiments can perform at least one of arbitrary subjective inspections or arbitrary objective measurements. In the subjective inspections, information (optotypes, etc.) is presented to the examinee, and the result is obtained based on a response to the information from the examinee. Examples of the subjective inspection include a visual field test, and a subjective refractivity measurement such as a distant test, a reading test, a contrast test, a glare test. In the objective measurements, light is projected onto an eye to be examined and information on the eye to be examined is acquired based on detection result(s) of returning light thereof. The objective measurements include a measurement for acquiring the characteristics of the eye to be examined and a photographing for acquiring an image of the eye to be examined. Examples of the objective measurement include an objective refractometry, a corneal shape measurement, an intraocular pressure measurement, a fundus photographing, a tomographic imaging using optical coherence tomography (hereinafter, OCT) (OCT imaging), and a measurement using OCT.

Hereafter, it is assumed that the ophthalmic apparatus according to the embodiments is an apparatus that can perform subjective inspection such as distant test, and reading test, and can also perform objective measurement such as objective refractometry using wavefront aberration measurement and corneal shape measurement. However, the configuration of the ophthalmic apparatus according to the embodiments is not limited to this.

### [Configuration]

The ophthalmic apparatus according to the embodiments includes a face support unit fixed to a base, and a stage movable in front, back, upward, downward, left, and right directions relative to the base. The stage is provided with a head unit in which an optical system for performing inspection (measurement) of the eye to be examined is housed. The face support unit and the head unit can be relatively moved by operating with respect to an operation unit provided on the side of an examiner. Further, in the ophthalmic apparatus, the face support unit and the head unit can be relatively moved automatically by performing alignment described below.

### (Optical system)

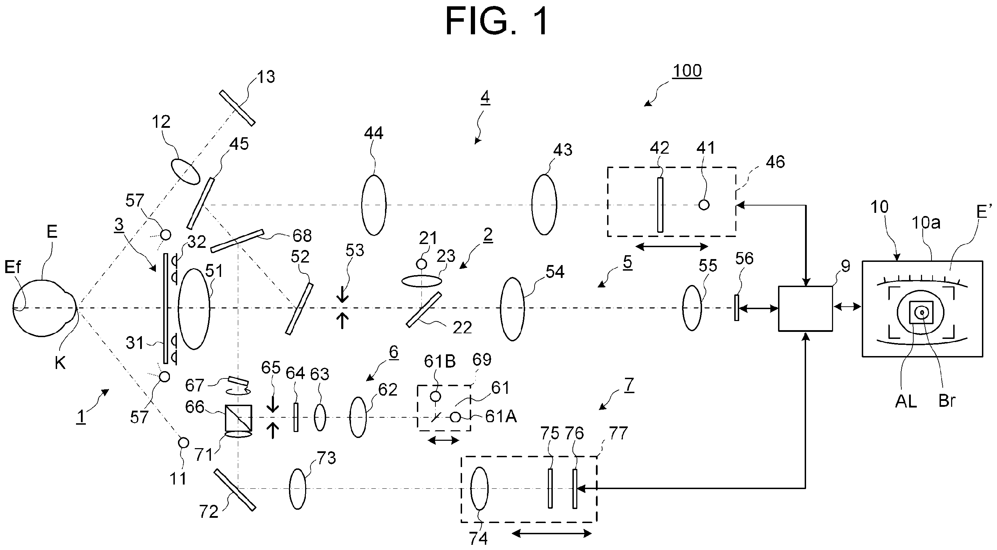

FIG. 1 shows an example of a configuration of an optical system of the ophthalmic apparatus according to the embodiments. The ophthalmic apparatus 100 according to the embodiments includes, as an optical system for performing inspections of an eye E to be examined, a Z alignment system 1, an XY alignment system 2, a keratometry system 3, an optotype projection system 4, an observation system 5, an aberration measurement projection system 6, and an aberration measurement light reception system 7. In addition, the ophthalmic apparatus includes a processor 9.

### (Processor 9)

The processor 9 controls each part of the ophthalmic apparatus. Further, the processor 9 is capable of performing various types of arithmetic processing. The processor 9 includes a processor. The processor 9 realizes the functions according to the embodiments, for example, by reading out computer program(s) stored in a storage circuit or a storage device and executing the computer program(s).

### (Observation System 5)

The observation system 5 is configured to photograph a moving image of an anterior segment of the eye E to be examined. For example, returning light from the anterior segment of the eye E to be examined, which is illuminated by light (for example, infrared light with a central wavelength of 950 nm) from a plurality of anterior segment illumination light sources 57 arranged at positions away from an optical axis of the observation system 5, passes through an objective lens 51, is transmitted through a dichroic mirror 52, and passes through an opening of a diaphragm 53. The light that has passed through the opening of the diaphragm 53 is transmitted through a half mirror 22, passes through a relay lens 54, and is guided to an imaging lens 55. The imaging lens 55 forms an image of the light, that has been guided from the relay lens 54, on a light receiving surface of an area sensor (image sensor) 56. The light receiving surface of the area sensor 56 is disposed at a position substantially conjugate optically to a pupil of the eye E to be examined. The area sensor 56 performs photographing and a signal outputting at a predetermined rate. The output (video signal) of the area sensor 56 is input to the processor 9. The processor 9 displays an anterior segment image E' based on this video signal on a display screen 10a of a display unit 10. The anterior segment image E' is an infrared moving image, for example.

### (Z alignment system 1)

The Z alignment system 1 is configured to project light (infrared light) for performing alignment in an optical axis direction (front-back directions, Z direction) of the observation system 5 onto the eye E to be examined. Light emitted from a Z alignment light source 11 is irradiated onto a cornea K of the eye E to be examined, is reflected on the cornea K, and is guided to an imaging lens 12. The imaging lens 12 forms an image of the light, that has been guided, on a light receiving surface of a line sensor 13. When a position of a corneal apex changes in the front-back directions, a projected position of the light onto the line sensor 13 changes. The output of the line sensor 13 is input to the processor 9. The processor 9 obtains the position of the corneal apex of the eye E to be examined based on the projected position of the light on the line sensor 13, and performs Z alignment based on this position.

### (XY alignment system 2)

The XY alignment system 2 is configured to irradiate light (infrared light) for performing alignment in directions (left-right directions (X direction), up-down directions (Y direction)) orthogonal to the optical axis direction of the observation system 5 onto the eye E to be examined. The XY alignment system 2 includes an XY alignment light source 21 disposed on an optical path that is branched from the observation system 5 by the half mirror 22. Light emitted from the XY alignment light source 21 passes through the relay lens 23, and is reflected by the half mirror 22. The light that has been reflected by the half mirror 22 is focused on a front focal position of the objective lens 51 on the optical axis of the observation system 5. The focused light is transmitted through the dichroic mirror 52, is made into collimated light by the objective lens 51, and is irradiated onto the cornea K of the eye E to be examined. The light reflected on a surface of the cornea K forms a Purkinje image near a reflection focal point on the corneal surface of the eye E to be examined. The XY alignment light source 21 is disposed at a position substantially conjugate optically to the focal position of the objective lens 51. Reflected light from the cornea K is guided to the area sensor 56 through the observation system 5. On the light receiving surface of the area sensor 56, an image Br, that is caused by the Purkinje image (bright spot) of the light emitted from the XY alignment light source 21, is formed.

The processor 9 displays an alignment mark AL and the anterior segment image E' including the bright spot image Br on the display screen 10a, as shown in FIG. 1. In case of performing XY alignment manually, an examiner performs a movement operation of the optical system so as to guide the bright spot image Br into the alignment mark AL. In case of performing XY alignment automatically, the processor 9 controls a mechanism for moving the optical system so as to cancel a displacement of the bright spot image Br with respect to the alignment mark AL.

### (Keratometry system 3)

The keratometry system 3 is configured to project a ring-shaped light flux (infrared light) for measuring a curvature of the cornea K onto the cornea K. A keratometry plate 31 is disposed in the vicinity of the objective lens 51. A keratometry ring light source 32 is provided on the back side (the objective lens 51 side) of the keratometry plate 31. By illuminating the keratometry plate 31 with light from the keratometry ring light source 32, the ring-shaped light flux is projected onto the cornea K. Reflected light (keratometry ring image) of the ring-shaped light flux) is detected by the area sensor 56 along with the anterior segment image. The processor 9 calculates a corneal shape parameter, by performing a known calculation based on this keratometry ring image. A placido ring plate consisting of a plurality of rings may be disposed instead of the keratometry ring. In this case, not only the curvature of the cornea, but also a corneal shape can be measured.

### (Optotype projection system 4)

The optotype projection system 4 is configured to present various kinds of optotypes such as a fixation target and an optotype for a subjective inspection to the eye E to be examined. An optotype chart 42 displays a pattern representing an optotype, under the control from the processor 9. Light (visible light) emitted from a light source 41 passes through the optotype chart 42, passes through a relay lens 43 and a field lens 44, is reflected by a reflective mirror 45, is transmitted through a beam splitter 68, and is reflected by the dichroic mirror 52. Light reflected by the dichroic mirror 52 passes through the objective lens 51 and is projected onto the fundus Ef.

A movement unit 46 including the light source 41 and the optotype chart 42 is movable along an optical axis of the optotype projection system 4. A position of the movement unit 46 is adjusted so that the optotype chart 42 and the fundus Ef are substantially conjugate optically to each other.

The optotype chart 42 can display the pattern representing the fixation target for fixating the eye E to be examined under the control from the processor 9. A fixation position can be moved by sequentially changing a display position of the pattern representing the fixation target in the optotype chart 42, a visual line of the eye to be examined can be guided, and/or an accommodation of the eye to be examined can be induced. Examples of such optotype chart 42 include an electronic display device using a liquid crystal panel or an electroluminescence (EL), and a device (turret type) that places any one of a plurality of optotypes drawn on a rotating glass plate, etc. on the optical axis as appropriate. Further, the optotype projection system 4 may include a glare test optical system for projection glare light onto the eye E to be examined along with the optotype described above.

In case of performing the subjective inspection, the processor 9 controls the movement unit 46 based on the result of the objective measurement. The processor 9 causes the optotype selected by the examiner or the processor 9 to be displayed on the optotype chart 42. Thereby, the optotype is presented to the examinee. The examinee responses with respect to the optotype. Upon receiving input of the response contents, the processor 9 performs further control or calculates a subjective inspection value. For example, in the visual acuity measurement, the processor 9 selects a next optotype based on the response to the Landolt ring or the like, presents the next optotype to the eye to be examined, and determines the visual acuity value by repeatedly performing this.

In the objective measurement (objective refractometry, etc.), a landscape chart is projected on the fundus Ef. Alignment is performed while causing the examinee to fixate the landscape chart, and the dioptric power is measured in a state where fogging is promoted.

### (Aberration measurement projection system 6, Aberration measurement light reception system 7)

The aberration measurement projection system 6 and the aberration measurement light reception system 7 are used for the measurement of the ocular aberration characteristics of the eye E to be examined. The aberration measurement projection system 6 is configured to project light flux (mainly, infrared light) for the measurement of the ocular aberration characteristics onto the fundus Ef. The aberration measurement light reception system 7 is configured to receive returning light of the light flux from the fundus Ef of the eye E to be examined. The ocular aberration characteristics of the eye E to be examined are obtained from light receiving result of the returning light acquired by the aberration measurement light reception system 7.

The aberration measurement projection system 6 includes a light source 61 that can output light in two or more wavelength regions with different central wavelengths. The light source 61 may be configured with a single light source that can change the wavelength region (central wavelength) of the output light. Alternatively, the light source 61 may be configured to switch between two or more light sources that output light with different wavelength regions (central wavelengths) from each other. In FIG. 1, it is assumed that the light source 61 includes a light source 61A that outputs light in a first wavelength region including a first central wavelength and a light source 61B that outputs light in a second wavelength region including a second central wavelength. For example, the first central wavelength is 560 nm (visible region) and the second central wavelength is 840 nm (near infrared region). In this case, the light source 61 outputs light from any one of the light source 61A and the light source 61B. In some embodiments, an optical path from the light source 61A and an optical path from the light source 61B are coupled by a dichroic mirror, and the light source 61A and the light source 61B are exclusively controlled to be turned on. In some embodiments, the optical path from the light source 61A and the optical path from the light source 61B are coupled by the dichroic mirror, a first shutter that can be inserted into or be removed is provided between the light source 61A and the dichroic mirror, and a second shutter that can be inserted into or be removed is provided between the light source 61B and the dichroic mirror.

For each of the light sources (point light sources) 61A and 61B, a light source that emits a minute point-like light is used. Examples of the light sources 61A and 61B include, for example, a SLD (Super Luminescent Diode) with high converging performance. However, an LD (laser diodes) with high converging performance or an LED with small emission diameter and high luminance may also be used.

The movement unit 69 including the light source 61 is movable along an optical axis of the aberration measurement projection system 6. The light source 61 is disposed at a position substantially conjugate optically to the fundus Ef. Light (measurement light) emitted from the light source 61 passes through a relay lens 62 and a field lens 63, and is transmitted through a polarizer 64. The polarizer 64 transmits the s-polarized component alone among the polarized components of the light emitted from the light source 61. The light transmitted through the polarizer 64 passes through an opening of a diaphragm 65, is reflected by a polarization beam splitter 66 that reflects the s-polarized component, passes through a rotary prism 67, and is reflected by a beam splitter 68. Light reflected by the beam splitter 68 is reflected by the dichroic mirror 52, passes through the objective lens 51, and is projected onto the fundus Ef.

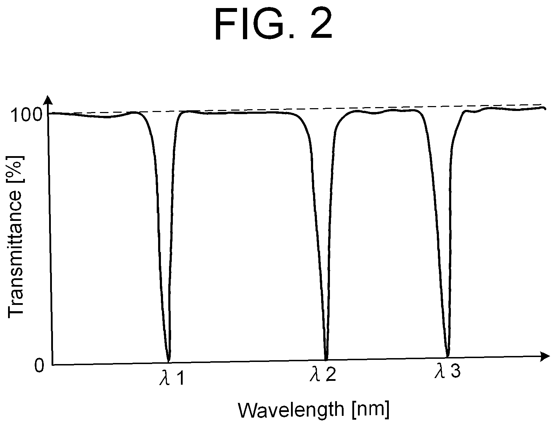

FIG. 2 schematically shows an example of wavelength selection characteristics of the beam splitter 68. **In** FIG. 2, a horizontal axis represents a transmittance of light, while the vertical axis represents wavelength.

For example, the beam splitter 68 reflects light in the wavelength region with the first wavelength λ1 as the central wavelength, light in the wavelength region with the second wavelength λ2 as the central wavelength, and light in the wavelength region with the third wavelength λ3 (0<λ1<λ2<λ3) as the central wavelength, and transmits light in other wavelength regions. For example, the first wavelength 11 is the central wavelength (560nm) of light emitted from the light source 61A, the second wavelength λ2 is the central wavelength (840nm) of light emitted from the light source 61B, and the third wavelength λ3 is the central wavelength (950nm) of light emitted from the anterior segment illumination light source 57.

Thereby, the beam splitter 68 can transmit the light from the optotype projection system 4, and can reflect the light from the light sources 61A and 61B in the aberration measurement projection system 6 and the returning light of the light from the light sources 61A and 61B. As a result, the wavelength separation between the optotype projection system 4, and the aberration measurement projection system 6 and aberration measurement light reception system 7 can be performed well. Such beam splitter 68 may be a mirror with wavelength selectivity disclosed in, for example, Japanese Unexamined Patent Application Publication No. 2010-099354.

It should be noted that, without placing a light source at the position of the light source 61, the light from the light source 61 may be guided to the relay lens 62 through an optical fiber that connects the light source and the ophthalmic apparatus. **In** this case, a fiber end of the optical fiber is disposed at a position substantially conjugate optically to the fundus Ef.

The rotary prism 67 is used for averaging the unevenness of reflectance on the blood vessels and/or the disease site of the fundus Ef, or for reducing the speckle noise caused by the SLD light source.

Light incident on the eye E to be examined no longer maintains its polarization state due to scattered reflection caused by the fundus. Therefore, the returning light from the fundus Ef becomes mixed light with the p-polarized component and the s-polarized component. Such returning light from the fundus Ef passes through the objective lens 51, and is reflected by the dichroic mirror 52 and the beam splitter 68. The returning light reflected by the beam splitter 68 passes through the rotary prism 67, and is guided to the polarization beam splitter 66. The polarization beam splitter 66 transmits the s-polarized component alone among the polarized components of the returning light. The p-polarized component of the light transmitted through the polarization beam splitter 66 passes through the field lens 71, is reflected by a reflective mirror 72, passes through a relay lens 73, and is guided to a movement unit 77. Light that is regularly reflected on a surface of the objective lens 51 or the cornea K of the eye E to be examined keeps the s-polarized. Therefore, the regularly reflected light is reflected by the polarization beam splitter 66, and does not enter the aberration measurement light reception system 7. Thereby, the occurrence of ghost can be reduced.

The movement unit 77 includes a collimator lens 74, a Hartmann plate 75, and an area sensor 76. The collimator lens 74 functions as a focusing lens through the movement of the movement unit 77. As the area sensor 76, for example, a CCD (Charge Coupled Device) image sensor or a CMOS (Complementary Metal Oxide Semiconductor) image sensor is used. Light that has been guided to the movement unit 77 passes through the collimator lens 74, and enters the Hartmann plate 75. The Hartmann plate 75 is disposed at a position optically conjugate to the pupil of the eye E to be examined. The movement unit 77 is movable along an optical axis of the aberration measurement light reception system 7. The movement unit 77 is moved along the optical axis according to the dioptric power of the eye E to be examined so that the fundus Ef and the front focal point of the collimator lens 74 are substantially conjugate optically to each other.

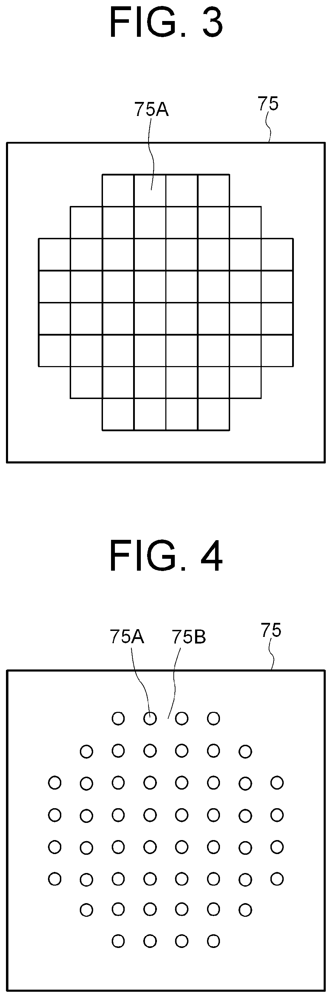

FIG. 3 and FIG. 4 show explanatory diagrams of the Hartmann plate 75 according to the embodiments. FIG. 3 and FIG. 4 schematically represent a configuration of the Hartmann plate 75 when viewed from the optical axis direction of the aberration measurement light reception system 7.

The Hartmann plate 75 generates a plurality of converging light from the returning light from the fundus Ef. As shown in FIG. 3 and FIG. 4, in the Hartmann plate 75, a plurality of microlenses 75A is arranged in a reticular pattern. The Hartmann plate 75 splits the incident light into many light fluxes, and then collects each of the light fluxes.

For example, the Hartmann plate 75 has a configuration in which the microlenses 75A are arranged on a glass plate by etching or molding, as shown in FIG. 3. **In** this case, the opening of each of the microlens can be larger, and the signal intensity can be increased.

Alternatively, as shown in FIG. 4, the Hartmann plate 75 may have a configuration in which the microlenses 75A are arranged with a light shielding portion 75B by forming a chromium light shielding film or the like around each microlens 75A. The microlenses 75A are not limited to those squarely arranged. The microlenses 75A may also be those concentrically arranged, those arranged at each apex position of a triangle, or those arranged in a hexagonal close-packed pattern.

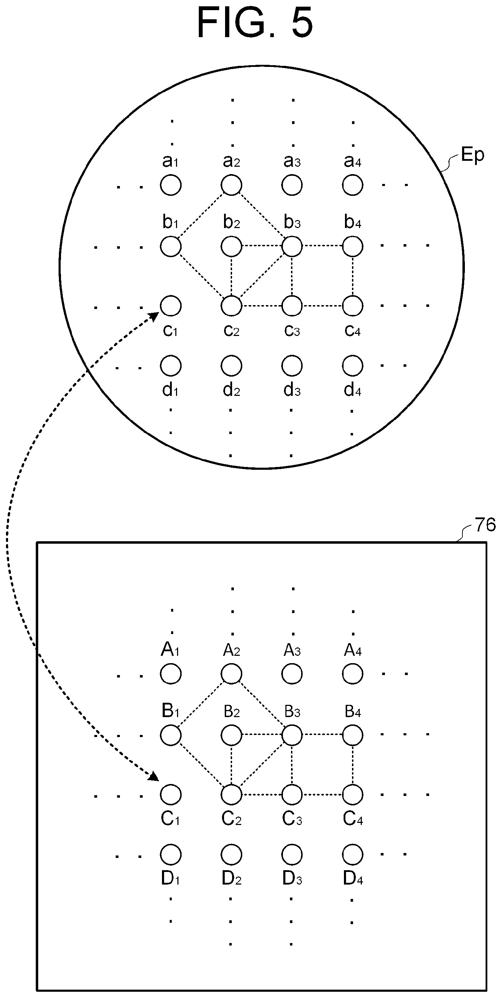

The area sensor 76 is disposed at the focal positions of the microlenses 75A, and detects light (converging light) that is respectively converged by the Hartmann plate 75. As shown in FIG. 5, on the light receiving surface of the area sensor 76, point images A₁, ..., B₁, ..., C₁, ... are formed by the microlenses 75A of the Hartmann plate 75 corresponding to irradiated regions a₁, ..., b₁, ..., c₁, ... of light on the pupil Ep of the eye E to be examined. **In** the case where the fundus Ef and the front focal point of the collimator lens 74 are in a substantially optically conjugate relationship as described above, intervals between the positions of the center of gravity of the point images formed on the light receiving surface of the area sensor 76 (or between peak positions of the brightness of the point images) are approximately equal to the distance between the lens centers of the microlenses 75A. The area sensor 76 detects a group of point images formed by microlenses 75A in the Hartmann plate 75. The processor 9 acquires detection signals based on the point image groups detected by area sensor 76 and the positional information indicating the detection positions of the point image groups, and analyzes the position of the point image formed by each microlens 75A to obtain the wavefront aberration of the light that has been entered into the Hartmann plate 75. Thereby, the ocular aberration characteristics of the eye E to be examined are obtained from the distances between the point images. The processor 9 obtains the dioptric power of the eye E to be examined from the obtained ocular aberration characteristics.

The processor 9 can move the movement unit 69 and the movement unit 77 in the optical axis direction, respectively, based on the calculated dioptric power, so that the light source 61 (light sources 61A and 61B), the fundus Ef, and the front focal position of the collimator lens 74 are conjugate optically to each other. Further, the processor 9 can move the movement unit 46 in the optical axis direction thereof, in conjunction with the movement of the movement units 69 and 77.

**In** some embodiments, the ophthalmic apparatus 100 can acquire transillumination images of the eye E to be examined. For example, the transillumination image can be acquired by turning one (or a part) of a plurality of anterior segment illumination light sources 57 on, projecting light onto the fundus Ef from a position away from the optical axis through the pupil, and receiving returning light of the light with the area sensor 56.

In some embodiments, the transillumination image is acquired by turning the XY alignment light source 21 on, projecting the light onto the fundus Ef through the pupil, and receiving the returning light of the light with the area sensor 56. In this case, the XY alignment light source 21 may be an SLD or a high-brightness LED. For example, the XY alignment light source 21 may be configured to output light from an LD as an alignment light source in performing alignment and to output light from the SLD or the high-brightness LED as an illumination light source for acquiring a transillumination image in acquiring transillumination images.

### (Configuration of processing system)

A processing system of the ophthalmic apparatus 100 according to the embodiments will be described.

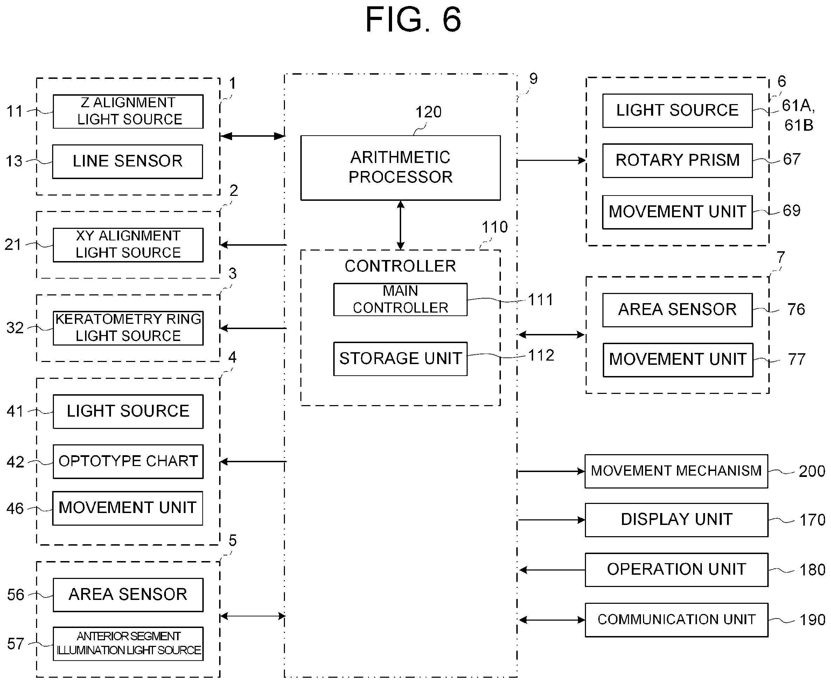

FIG. 6 shows an example of a functional configuration of a processing system of the ophthalmic apparatus 100. FIG. 6 illustrates an example of a functional block diagram of the processing system of the ophthalmic apparatus according to the embodiments. In FIG. 6, like parts are designated by like reference numerals as in FIG. 1 and repetitious description of such parts may not be provided.

The processor 9 includes a controller 110 and an arithmetic processor 120. Further, the ophthalmic apparatus 100 includes a display unit 170, an operation unit 180, a communication unit 190, and a movement mechanism 200.

The movement mechanism 200 is a mechanism for moving the head unit in front, back, upward, downward, left and right directions. Here, the head unit houses the optical systems such as the Z alignment system 1, the XY alignment system 2, the keratometry system 3, the optotype projection system 4, the observation system 5, the aberration measurement projection system 6, and the aberration measurement light reception system 7. For example, the movement mechanism 200 is provided with an actuator that generates a driving force for moving the movement mechanism 200 and a transmission mechanism that transmits the driving force from the actuator to the movement mechanism 200. The actuator includes a pulse motor, for example. The transmission mechanism includes a combination of gears, a rack and pinion, and the like, for example. The controller 110 (main controller 111) performs control for the movement mechanism 200 by sending a control signal to the actuator.

### (Controller 110)

The controller 110 includes a processor and controls each part of the ophthalmic apparatus. The controller 110 includes a main controller 111 and a storage unit 112. The storage unit 112 stores, in advance, a computer program for controlling the ophthalmic apparatus. The computer program includes program(s) for controlling light source, program(s) for controlling sensor, program(s) for controlling optical system, program(s) for arithmetic processing, program(s) for user interface, and the like. The main controller 111 operates according to the computer programs, and thereby the controller 110 performs the control process.

The main controller 111 performs various controls of the ophthalmic apparatus, as a measurement controller. Examples of control for the Z alignment system 1 include control of the Z alignment light source 11, control of the line sensor 13. Examples of the control of the Z alignment light source 11 include turning on and off of the light source, and adjustment of light quantity. Examples of the control of the line sensor 13 include adjustment of exposure of a detecting element, adjustment of gain of the detecting element, and adjustment of detecting rate of the detecting element. Thereby, the Z alignment light source 11 is switched between lighting and non-lighting or the light quantity is changed. The main controller 111 captures a signal detected by the line sensor 13 and identifies a projected position of light onto the line sensor 13 based on the captured signal. The main controller 111 obtains a position of a corneal apex of the eye E to be examined based on the identified projected position and controls the movement mechanism 200 based on the identified position to move the head unit in front and back directions (Z alignment).

Examples of control for the XY alignment system 2 include control of the XY alignment light source 21. Examples of the control of the Z alignment light source 21 include turning on and off of the light source, and adjustment of light quantity. Thereby, the XY alignment light source 21 is switched between lighting and non-lighting, or the light quantity is changed. The main controller 111 captures a signal detected by the area sensor 56, and identifies a position of a bright spot image on the basis of returning light of the light from the XY alignment light source 21 based on the captured signal. The main controller 111 controls the movement mechanism 200 to move the head unit in left, right, upward, downward directions so as to cancel a displacement the position of the bright spot image with respect to a predetermined target position (for example, a center position of the alignment mark) (XY alignment).

Examples of control for the keratometry system 3 include control of the keratometry ring light source 32. Examples of the control of the keratometry ring light source 32 include turning on and off of the light source, and adjustment of light quantity. Thereby, the keratometry ring light source 32 is switched between lighting and non-lighting, or the light quantity is changed. The main controller 111 controls the arithmetic processor 120 to perform a known calculation on a keratometry ring image detected by the area sensor 56. Thereby, corneal shape parameter(s) of the eye E to be examined is/are obtained.

Examples of the control for the optotype projection system 4 include control of the light source 41, control of the optotype chart 42, and movement control of the movement unit 46. Examples of the control for the light source 41 include turning the light source on and off, and adjustment of light quantity. Thereby, the light source 41 is switched between lighting and non-lighting, or the light quantity is changed. Examples of the control of the optotype chart 42 include displaying on and off of the optotypes and/or the fixation target, and switching the display position of the fixation target. Thereby, the optotypes and/or the fixation target are/is projected onto the fundus Ef of the eye E to be examined. For example, the optotype projection system 4 include a movement mechanism that moves the movement unit 46 in the optical axis direction. As is the case with the movement mechanism 200, this movement mechanism is provided with an actuator that generates driving force for moving the movement mechanism and a transmission mechanism that transmits the driving force from the actuator to the movement unit 46. The main controller 111 controls the movement mechanism by sending a control signal to the actuator to move the movement unit 46 in the optical axis direction. Thereby, a position of the movement unit 46 is adjusted so that the optotype chart 42 and the fundus Ef are conjugate optically to each other.

Examples of the control for the observation system 5 include control of the area sensor 56, and control of the anterior segment illumination light source 57. Examples of the control of the area sensor 56 include adjustment of exposure of the area sensor 56, adjustment of gain of the area sensor 56, and adjustment of detecting rate of the area sensor 56. The main controller 111 captures signal(s) detected by the area sensor 56, and controls the arithmetic processor 120 to perform processing such as forming images based on the captured signal(s). Examples of control of the anterior segment illumination light source 57 include turning on and off the light source, adjustment of light quantity. Thereby, the anterior segment illumination light source 57 is switched between lighting and non-lighting, one or a part of the anterior segment illumination light sources 57 is turned on, or the light quantity of each light source is changed.

Examples of control for the aberration measurement projection system 6 include control of the light sources 61A and 61B, control of the rotary prism 67, control of the movement unit 69. Examples of the control for the light sources 61A and 61B include turning the light sources on and off, and adjustment of light quantity. Thereby, the light sources 61A and 61B are switched between lighting and non-lighting, the light quantity is changed, the wavelength region of the light emitted from the light source 61 is changed. Examples of the control of the rotary prism 67 include control of rotating the rotary prism 67. For example, a rotary mechanism that rotates the rotary prism 67 is provided and the main controller 111 controls this rotary mechanism to rotate the rotary prism 67. For example, the aberration measurement projection system 6 include a movement mechanism that moves the movement unit 69 in the optical axis direction. As is the case with the movement mechanism 200, this movement mechanism is provided with an actuator that generates driving force for moving the movement mechanism and a transmission mechanism that transmits the driving force from the actuator to the movement unit 69. The main controller 111 controls the movement mechanism by sending a control signal to the actuator to move the movement unit 69 in the optical axis direction.

Examples of the control for the aberration measurement light reception system 7 include control of the area sensor 76, and movement control of the movement unit 77. Examples of the control of the area sensor 76 include adjustment of exposure of the area sensor 76, adjustment of gain of the area sensor 76, and adjustment of detecting rate of the area sensor 76. The main controller 111 captures signal(s) detected by the area sensor 76, and controls the arithmetic processor 120 to perform calculation processing of the ocular aberration characteristics based on the captured signal(s). For example, the aberration measurement light reception system 7 include a movement mechanism that moves the movement unit 77 in the optical axis direction. As is the case with the movement mechanism 200, this movement mechanism is provided with an actuator that generates driving force for moving the movement mechanism and a transmission mechanism that transmits the driving force from the actuator to the movement unit 77. The main controller 111 controls the movement mechanism by sending a control signal to the actuator to move the movement unit 77 in the optical axis direction. The main controller 111 controls the movement mechanism by sending a control signal to the actuator to move the movement unit 77 in the optical axis direction.

The main controller 111 can, as a display controller, display various kinds of information on the display unit 170. Examples of the information displayed on the display unit 170 include a result of the objective measurement (aberration measurement result) and a result of the subjective inspection acquired by using the above optical system, and an image or information based on these. For example, the dioptric power calculated by the arithmetic processor 120, or the like is displayed on the display unit 170. The main controller 111 can display these information for each area of the focal point distances of the IOL, or can identifiably display a part of the information. In some embodiments, the main controller 111 displays a wavefront aberration map representing the distribution of wavefront aberration, a simulation image representing the view, and the visual acuity simulation result(s) on the display unit 170.

Further, the main controller 111 performs a process of writing data in the storage unit 112 and a process of reading out data from the storage unit 112.

### (Storage unit 112)

The storage unit 112 stores various types of data. Examples of the data stored in the storage unit 112 include inspection result(s) of the subjective inspection, measurement result(s) of the objective measurement, image data of the anterior segment image, image data of the Hartmann point image, information on eye to be examined, and processing result(s) obtained by the arithmetic processor 120. The information on the eye to be examined includes information on the examinee such as patient ID and name, and information on the eye to be examined such as identification information of the left/right eye. Further, the storage unit 112 stores various types of programs and data to run the ophthalmic apparatus.

### (Arithmetic processor 120)

The arithmetic processor 120 includes a processor and executes the processing of each of the following parts according to the program(s) stored in a storage unit (not shown) (or storage unit 112).

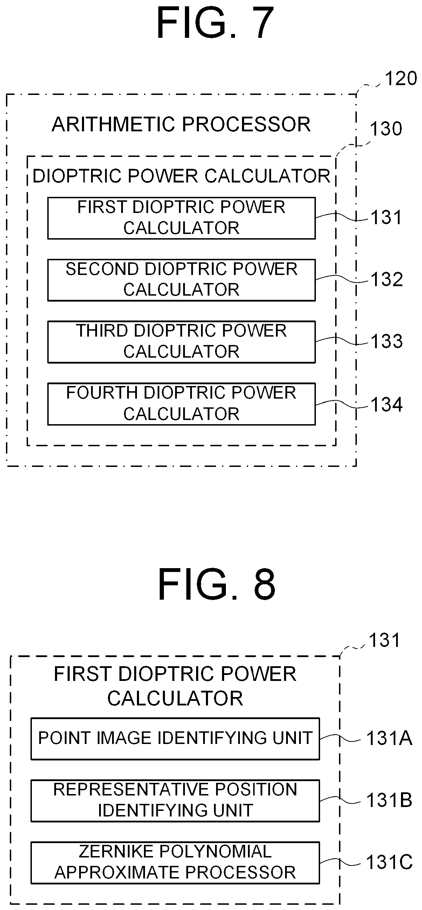

FIG. 7 shows a functional block diagram of an example of a configuration of the arithmetic processor 120 in FIG. 6.

The arithmetic processor 120 includes a dioptric power calculator 130.

### (Dioptric power calculator 130)

The dioptric power calculator 130 obtains the dioptric power of the eye E to be examined using various calculation processing methods. The dioptric power calculator 130 includes a first dioptric power calculator 131, a second dioptric power calculator 132, a third dioptric power calculator 133, and a fourth dioptric power calculator 134.

The first dioptric power calculator 131 performs, as a monofocal dioptric power calculator, monofocal type calculation processing that calculates the dioptric power of the eye E to be examined using a calculation processing method corresponding to the monofocal type IOL, for example. The second dioptric power calculator 132 performs, as a multifocal type dioptric power calculator, multifocal refractive type calculation processing that calculates the dioptric power of the eye E to be examined using a calculation processing method corresponding to the multifocal refractive type IOL, for example. The third dioptric power calculator 133 performs, as a multifocal diffractive type dioptric power calculator, multifocal diffractive type calculation processing that calculates the dioptric power of the eye E to be examined using a calculation processing method corresponding to the multifocal diffractive type IOL, for example. The fourth dioptric power calculator 134 performs, as an EDoF type dioptric power calculator, EDoF type calculation processing that calculates the dioptric power of the eye E to be examined using a calculation processing method corresponding to the EDoF type IOL, for example.

The functional blocks that make up the first dioptric power calculator 131, the second dioptric power calculator 132, the third dioptric power calculator 133, and the fourth dioptric power calculator 134 may be shared as appropriate, in case that they have the same function.

### (First dioptric power calculator 131)

The first dioptric power calculator 131 calculates a single dioptric power based on the wavefront aberration information including the Hartmann image acquired by the wavefront aberration measurement.

FIG. 8 shows a functional block diagram of an example of a configuration of the first dioptric power calculator 131 shown in FIG. 7.

The first dioptric power calculator 131 includes a point image identifying unit 131A, a representative position identifying unit 131B, and a Zernike polynomial approximate processor 131C.

The point image identifying unit 131A identifies the point images that make up the Hartmann image. The point image identifying unit 131A identifies the point images based on the brightness values of the Hartmann image acquired by the area sensor 56. In some embodiments, enhancement processing of the point image(s) is performed on the Hartmann image, before the point images are identified by the point image identifying unit 131A.

The representative position identifying unit 131B identifies a representative position of the point image identified by the point image identifying unit 131A. Examples of the representative position include a position of the center of gravity, a center position, a position in the point image closest to the center of the Hartmann image, and a position in the point image farthest from the center of the Hartmann image. In the present embodiment, the representative position identifying unit 131B identifies the position of the center of gravity, as the representative position.

The Zernike polynomial approximate processor 131C performs Zernike polynomial approximate processing based on the representative positions of the point images identified by the representative position identifying unit 131B, and obtains the spherical power S, the astigmatic power C and the astigmatic axis angle A as the single dioptric power. In other words, the Zernike polynomial approximate processor 131C obtains slopes of light beams at the representative positions of the point images identified by the representative position identifying unit 131B, and obtains an approximate expression for the wavefront by a known calculation using the obtained amounts of the slopes of the light beams. The obtained approximate expression for the wavefront is expressed by Zernike coefficients and a Zernike polynomial. The wavefront aberration information is represented by the Zernike coefficients. In this case, the Zernike polynomial approximate processor 131C can normalize the wavefront aberration information using the pupil diameter of the eye E to be examined or the pupil diameter of the schematic eye, as disclosed in Japanese Unexamined Patent Application Publication No. 2002-209854, for example. The Zernike polynomial approximate processor 131C obtains the spherical power S, the astigmatic power C and the astigmatic axis angle A from the low-order term(s) of the Zernike coefficients, using a known calculation. For example, the Zernike polynomial approximate processor 131C can calculate the dioptric power using a method disclosed in Japanese Unexamined Patent Application Publication No. 2002-209854 or Japanese Unexamined Patent Application Publication No. 2017-213124.

### (Second dioptric power calculator 132)

The second dioptric power calculator 132 calculates a plurality of dioptric powers, each of which corresponds to each of a plurality of focal point distances of the IOL, based on the wavefront aberration information including the Hartmann image acquired by the wavefront aberration measurement. In other words, the second dioptric power calculator 132 calculates a plurality of dioptric powers including the dioptric power corresponding to a far point and the dioptric power corresponding to a near point, for each area corresponding to the focal point distance of the IOL.

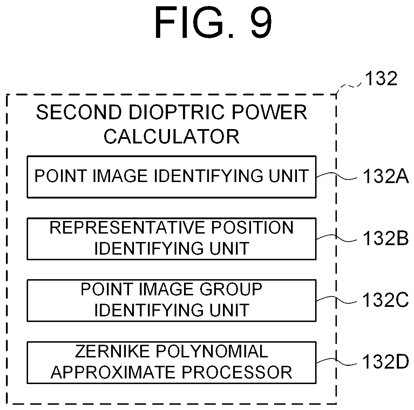

FIG. 9 shows a functional block diagram of an example of a configuration of the second dioptric power calculator 132 shown in FIG. 7.

The second dioptric power calculator 132 includes a point image identifying unit 132A, a representative position identifying unit 132B, and a point image group identifying unit 132C, and a Zernike polynomial approximate processor 132D.

The point image identifying unit 132A identifies the point images that make up the Hartmann image, in the same way as the point image identifying unit 131A. In some embodiments, enhancement processing of the point image(s) is performed on the Hartmann image, before the point images are identified by the point image identifying unit 132A.

The representative position identifying unit 132B identifies a representative position (position of the center of gravity) of the point image identified by the point image identifying unit 132A, in the same way as the representative position identifying unit 131B.

The point image group identifying unit 132C classifies two or more separated point images, each of which is generated by separating the point image that should has originally formed, into point image groups for each focal point distance of the IOL (according to focal point of distance of the IOL).

The Zernike polynomial approximate processor 132D calculates the dioptric power of the eye E to be examined in the same way as the Zernike polynomial approximate processor 131C, for each point image group identified by the point image group identifying unit 132C.

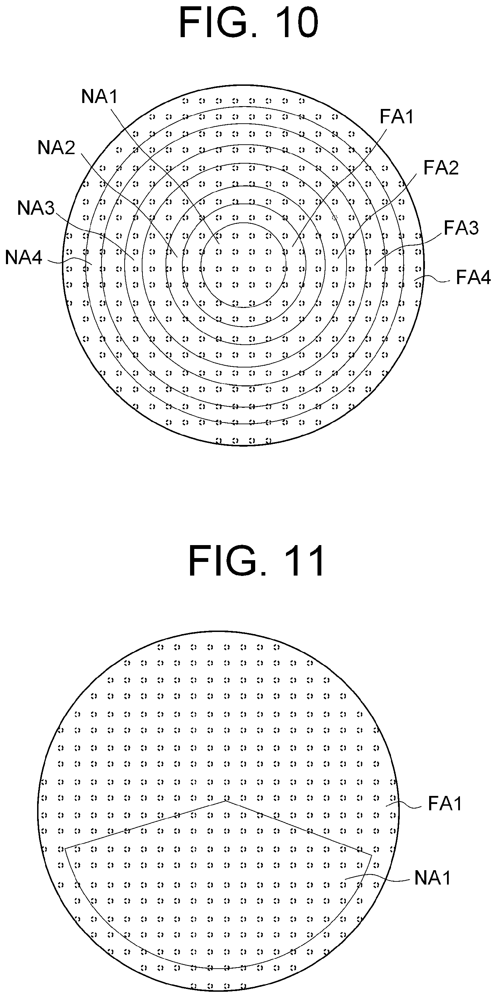

FIG. 10 and FIG. 11 schematically show point images acquired by the area sensor 56, each of the point images corresponding to the lens areas of the multifocal refractive type IOL according to the embodiments. FIG. 10 represents the point images corresponding to a toric (annular) type multifocal refractive type IOL. In the toric type, areas with different focal point distances are arranged concentrically. FIG. 11 represents the point images corresponding to a sector type multifocal refractive type IOL. In the sector type, areas with different focal point distances are arranged below the lens area.

In the case that the number of the focal points of the toric type multifocal refractive type IOL is "2", for example, near (near vision, reading) areas, which can focus on near objects, and far (far vision, distant) areas, which can focus on the distance, are arranged alternately, from the center to the outside. In FIG. 10, from the center to the outside, a near area NA1, a far area FA1, a near area NA2, a far area FA2, ..., a near area NA4, and a far area FA4 are arranged alternately. In the case that the number of the focal points of the toric type multifocal refractive type IOL is "3", similarly, the near areas, medium (medium vision) areas, which can focus on intermediate distance between the near object and the distance, and the far areas are arranged in order, from the center to the outside. In the case that the number of the focal points of the toric type multifocal refractive type IOL is 4 or more, similarly, the near areas, two or more medium areas with intermediate distances different from each other, and the far areas are arranged in order, from the center to the outside. In the present example, the center side is arranged as the near area and the area far from the center is arranged as the far area. However, the center side may be arranged as the far area and the area far from the center may be arranged as the near area.

The second dioptric power calculator 132 identifies the point image group included in the area corresponding to a predetermined focal point distance, and calculates the dioptric powers for each point image group, based on the wavefront aberration information obtained from the identified point image groups.

In the case that the number of the focal points of the sector type multifocal refractive type IOL is "2", as shown in FIG. 11, a near area NA1 is arranged below the lens area where the entire area is a far area FA1. In the case that the number of the focal points of the sector type multifocal refractive type IOL is 3 or more, for example, each of one or more medium areas are arranged between the far area FA1 and the near area NA1.

In this case, the second dioptric power calculator 132 identifies the point image group included in the area corresponding to a focal point distance, and calculates the dioptric powers for each point image group, based on the wavefront aberration information obtained from the identified point image groups, in the same way as the toric type.

Thereby, for example, the dioptric power can be calculated from the point image group(s) included in the near area, and the dioptric power can be calculated from the point image group(s) included in the far area. In some embodiments, the dioptric power can be calculated from the point image groups, each of which is included in each of the one or more medium areas.

### (Third dioptric power calculator 133)

The third dioptric power calculator 133 calculates a plurality of dioptric powers, each of which corresponds to each of a plurality of focal point distances of the IOL, based on the wavefront aberration information including the Hartmann image acquired by the wavefront aberration measurement. Specifically, the third dioptric power calculator 133 classifies the two or more separated point images, each of which is generated by separating the point image that constitutes the Hartmann image, into the point image groups for each focal point distance of the IOL, and calculates a plurality of dioptric powers including a dioptric power corresponding to the far point and a dioptric power corresponding to the near point for each of focal point distances based on the classified two or more separated point images.

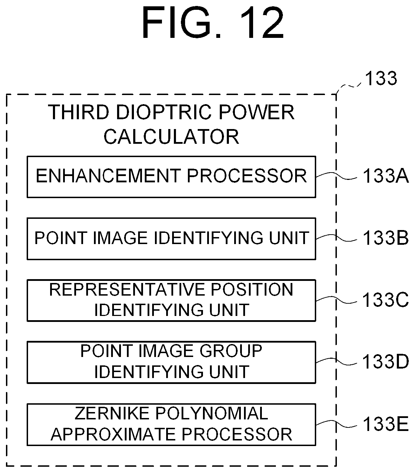

FIG. 12 shows a functional block diagram of an example of a configuration of the third dioptric power calculator 133 shown in FIG. 7.

The third dioptric power calculator 133 includes an enhancement processor 133A, a point image identifying unit 133B, a representative position identifying unit 133C, a point image group identifying unit 133D, and a Zernike polynomial approximate processor 133E.

The enhancement processor 133A performs enhancement processing of point image in the Hartmann image. For example, the enhancement processor 133A increases the contrast of the Hartmann image and removes portions where the brightness values are saturated.

The point image identifying unit 133B identifies the point images that constitute the Hartmann image, in the same way as the point image identifying unit 131A. Specifically, the point image identifying unit 133B identifies the two or more separated point images, each of which is generated by separating the point image that constitutes the Hartmann image. **In** this case, the point image identifying unit 133B identifies one or more separated point images on the far point side by analyzing the Hartmann image, and identifies the remaining one or more separated point images on the near point side by analyzing the Hartmann image on which the enhancement processing has been performed by the enhancement processor 133A. **In** other words, the point image identifying unit 133B identifies point images with a predetermined first brightness value or greater from the Hartmann image as the separated point images on the far point side, and identifies point images with a predetermined second brightness value or greater in the Hartmann image from which the portions with the saturated brightness values are removed by increasing contrast as the separated point images on the near point side. Thereby, the separated point images on the near point side, where the contrast is reduced caused by the multifocal diffractive type **IOL** can be easily identified.

**In** some embodiments, the point image identifying unit 133B identifies one or more separated point images on the far point side and the one or more separated point images on the near point side by analyzing the Hartmann image on which the enhancement processing has been performed by the enhancement processor 133A.

**In** some embodiments, the wavefront aberration measurement is performed using light in the visible region (for example, light from light source 61A) in order to facilitate identification of the separated point images on the near point side.

The representative position identifying unit 133C identifies a representative position (position of the center of gravity) of the point image identified by the point image identifying unit 133B, in the same way as the representative position identifying unit 131B.

The point image group identifying unit 133D classifies the two or more separated point images, which are identified by the representative position identifying unit 133C, into any one of two or more point image groups corresponding to the focal point distances of the **IOL.** The point image group identifying unit 133D classifies each of the two or more separated point images, from which one point image is separated, into any one of a point image group of the separated point images of the near point, a point image group of one or more separated point images corresponding to one or more medium point, and a point image group of the separated point images of the far point, in order from closest to the center of the Hartmann image.

The Zernike polynomial approximate processor 133E calculates the dioptric power of the eye E to be examined in the same way as the Zernike polynomial approximate processor 131C, for each point image group identified by the point image group identifying unit 133D.

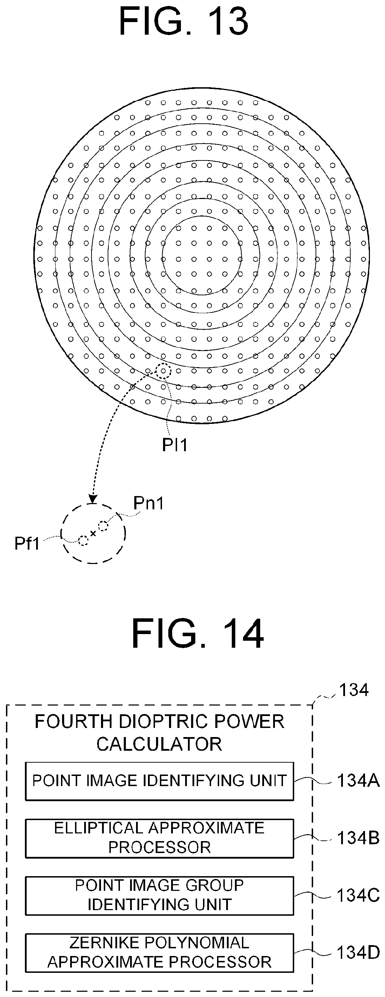

FIG. 13 schematically shows point images acquired by the area sensor 56, each of the point images corresponding to the lens areas of the multifocal diffractive type IOL according to the embodiments. In FIG. 13, the number of the focal points is assumed to be "2".

The point image that constitutes the Hartmann image is separated into the two or more separated point images according to the focal point distance of the IOL. As shown in FIG. 13, when the number of focal points is "2", the point image PI1 is separated into a separated point image (point image at near point) Pn1 closer to the center of the Hartmann image and a separated point image (point image at far point) Pf1 farther from the center of the Hartmann image, with reference to the point image that should originally be formed. Depending on the configuration of the optical system, the point image PI1 may be separated such that the separated point image close to the center of the Hartmann image is point image Pf1 at far point, and a separated point image far from the center of the Hartmann image is a point image Pn1 at near point.

The third dioptric power calculator 133 classifies the identified two or more separated point images into any one of the two or more point image groups corresponding to the focal point distances of the IOL, and calculates the dioptric power for each point image group. In FIG. 13, the point image group identifying unit 133D classifies the two separated point image, each of which corresponds to each point image that makes up the Hartmann image, into the point image group of the near point and the point image group of the far point. The Zernike polynomial approximate processor 133E calculates the dioptric power for each point image group, based on the wavefront aberration information obtained from the classified point image groups. The third dioptric power calculator 133 can calculate the dioptric power for each point image group as described above, for each Hartmann image.

### (Fourth dioptric power calculator 134)

The fourth dioptric power calculator 134 identifies the two separated point images corresponding to the point images that constitute the Hartmann image based on the wavefront aberration information including the Hartmann image acquired by the wavefront aberration measurement, classifies the identified separated point images into any one of the two point image groups, and calculates the dioptric power for each point image group based on the wavefront aberration information obtained from the classified point image groups. Specifically, the fourth dioptric power calculator 134 classifies two focal points of an approximate ellipse, which is identified by performing elliptical approximation on each of a plurality of point images that constitute the Hartmann image, into groups for each focal point distance, and calculates a plurality of dioptric powers including a dioptric power corresponding to the far point and a dioptric power corresponding to the near point for each of focal point distances based on the classified two or more separated point images.

FIG. 14 shows a functional block diagram of an example of a configuration of the fourth dioptric power calculator 134 shown in FIG. 7.

The fourth dioptric power calculator 134 includes a point image identifying unit 134A, an elliptical approximate processor 134B, a point image group identifying unit 134C, and a Zernike polynomial approximate processor 134D.

The point image identifying unit 134A identifies the point images that make up the Hartmann image, in the same way as the point image identifying unit 131A. In some embodiments, enhancement processing of the point image(s) is performed on the Hartmann image, before the point images are identified by the point image identifying unit 134A.

The elliptical approximate processor 134B identifies a plurality of approximate ellipses by performing know elliptical approximate processing on each of the plurality of point images identified by the point image identifying unit 134A, and identifies the two focal points of each of the plurality of identified approximate ellipses.

The point image group identifying unit 134C identifies the focal point closer to the center of the Hartmann image among the two focal points of the approximate ellipse identified by the elliptical approximate processor 134B as the point image at near point and the focal point farther from the center of the Hartmann image as the point image at far point. The point image group identifying unit 134C classifies the plurality of point images at near point and the plurality of point images at far point, which are identified for each of the plurality of approximate ellipses, into a point image group of the point image at near point and a point image group of the point image at far point.

The Zernike polynomial approximate processor 134D calculates the dioptric power of the eye E to be examined in the same way as the Zernike polynomial approximate processor 131C, for each point image group identified by the point image group identifying unit 134C.

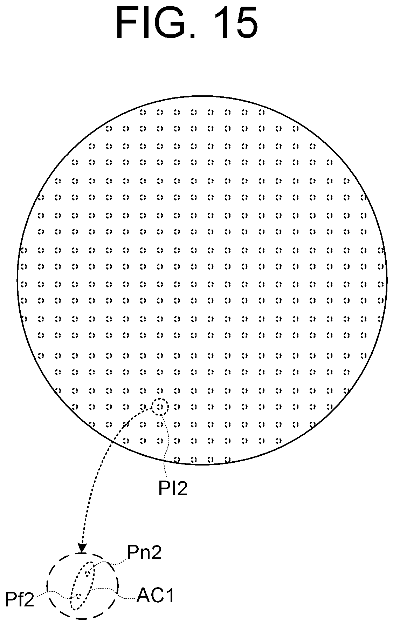

FIG. 15 schematically shows point images acquired by the area sensor 56, each of the point images corresponding to the lens areas of the EDoF type IOL according to the first embodiment.

The point image identifying unit 134A identifies the point images by analyzing the Hartmann image. In this case, the point image that constitutes the Hartmann image is an image that extends in a direction connecting the far point and the near point (blurred image) due to the deep depth of focus.

In the case where the point images are arranged in a toric shape or a sector shape similar to the multifocal refractive type IOL, the arithmetic processor 120 can calculate the dioptric power for each point image group similar to the multifocal refractive type IOL in the second dioptric power calculator 132. In the case where the point image is separated into the two or more separated point images similar to the multifocal diffractive type IOL, the arithmetic processor 120 can calculate the dioptric power for each point image group similar to the multifocal diffractive type IOL in the third dioptric power calculator 133.

In the case where a shape of the point image is an elliptical shape, the elliptical approximate processor 134B performs elliptical approximate processing on a shape of the point image PI2 to identify an approximate ellipse AC1, and identifies two focal points Pn2 and Pf2 of the identified approximate ellipse AC1. The point image group identifying unit 134C identifies the focal point Pn2 close to the center of the Hartmann image among the identified two focal points as the point image at near point and the focal point Pf2 far from the center of the Hartmann image as the point image at far point. Further, the point image group identifying unit 134C similarly classifies two focal points as the point image at near point and the point images at far point for each of the plurality of point images, and classifies the identified point images at near point and the identified point images at far point into a point image group of the point image at near point and a point image group of the point image at far point. The Zernike polynomial approximate processor 134D calculates the dioptric power for each point image group, based on the wavefront aberration information obtained from the classified point image groups.

Further, the dioptric power calculator 130 calculates a corneal dioptric power, a corneal astigmatism power, and a corneal astigmatic axis angle based on the keratometry ring image acquired by the observation system 5. For example, the dioptric power calculator 130 calculates a corneal curvature radius of the steepest meridian and/or the flattest meridian of the anterior surface of the cornea by analyzing the keratometry ring image and calculates above parameters based on the corneal curvature radius.

### (Display unit 170, Operation unit 180)

The display unit 170 displays information upon receiving control of the controller 110 (main controller 111), as an interface unit. The display unit 170 includes the display unit 10 shown in FIG. 1.

The display unit 170 can receive control from the controller 110 (main controller 111) as a display controller, and can display result(s) of processing performing by the arithmetic processor 120. Examples of result(s) of processing performed by the arithmetic processor 120 include one or more dioptric powers calculated by the dioptric power calculator 130, the wavefront aberration information such as wavefront aberration maps, and simulation results of views or acuity values.

Examples of the one or more dioptric powers calculated by the dioptric power calculator 130 include the single dioptric power calculated by the first dioptric power calculator 131, the dioptric powers, which are calculated by the second dioptric power calculator 132 or the third dioptric power calculator 133, for the number of focal points for each focal point distance (area) of the IOL, and the dioptric powers of the far point side and the near point side, which are calculated by the fourth dioptric power calculator 134. In some embodiments, the dioptric power of the far point side and the dioptric power of the near point side among the dioptric powers for the number of focal points calculated by the second dioptric power calculator 132 or the third dioptric power calculator 133 are displayed on the display unit 170. In some embodiments, a difference between the dioptric power of the farthest point side and the dioptric power of the nearest point side among the dioptric powers calculated by the fourth dioptric power calculator 134, is displayed on the display unit 170.

The operation unit 180 is used to operate the ophthalmic apparatus, as the user interface unit. The operation unit 180 includes various types of hardware keys (the joystick, buttons, switches, etc.) provided in the ophthalmic apparatus. Further, the operation unit 180 may include various kinds of software keys (buttons, icons, menus, etc.) displayed on the touch panel type display screen.

At least part of the display unit 170 and the operation unit 180 may be integrally configured. A typical example of this is the touch-panel display screen 10a.

### (Communication unit 190)

The communication unit 190 has the function of communicating with an external device (not shown). The communication unit 190 may be provided in the processor 9, for example. The communication unit 190 has a configuration corresponding to the mode of communication with the external device.

The arithmetic processor 120 is an example of the "ophthalmic information processing apparatus" according to the embodiments. The communication unit 190, or the aberration measurement projection system 6 and aberration measurement light reception system 7 is an example of the "acquisition unit" according to the embodiments. The aberration measurement projection system 6 and the aberration measurement light reception system 7 are an example of the "measurement optical system" according to the embodiments. The dioptric power calculator 130 is an example of the "calculator" according to the embodiments. The controller 110 (main controller 111) is an example of the "display controller" according to the embodiments. The display unit 170 is an example of the "display means" according to the embodiments.

### [Operation Example]

Operation examples of the ophthalmic apparatus according to the embodiments will be described.

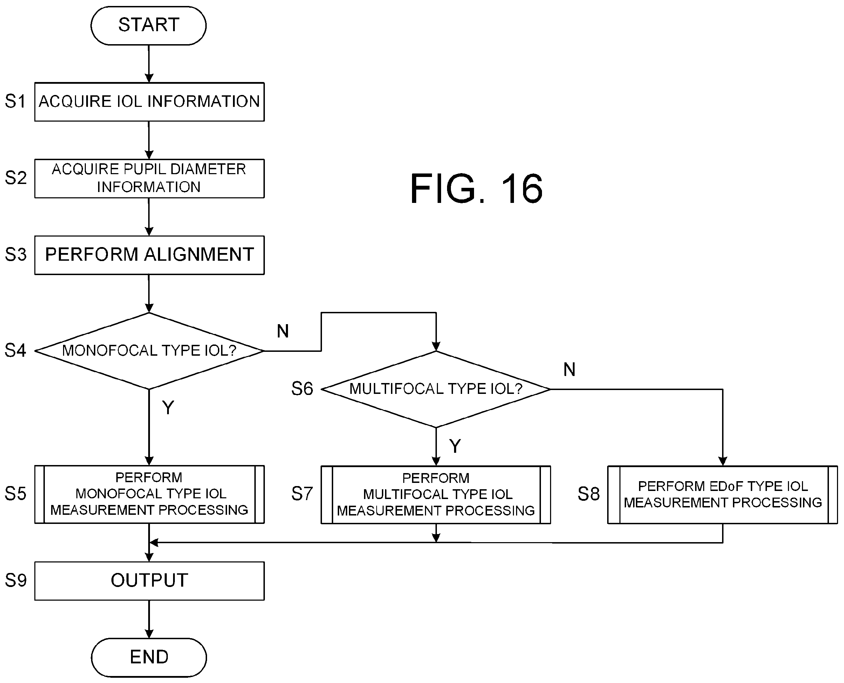

FIGS. 16 to 28 show flowcharts of examples of an operation of the ophthalmic apparatus 100 according to the embodiments.

FIG. 16 represents a flowchart of an example of an operation of the ophthalmic apparatus 100 that calculates the dioptric power of the eye E to be examined using a measurement processing method according to the type of IOL worn by the eye E to be examined.

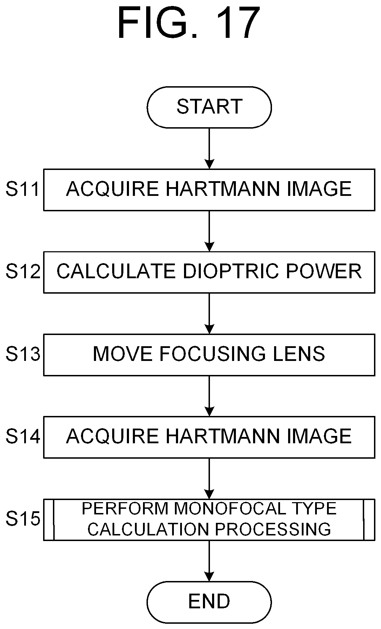

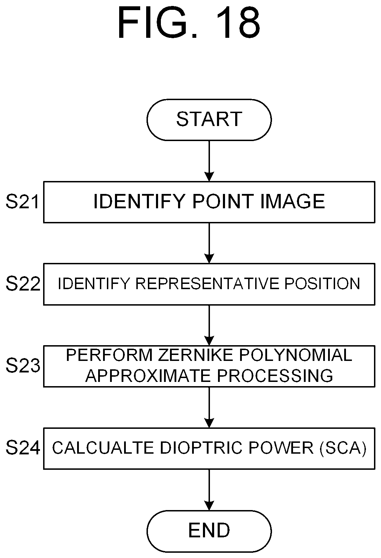

FIG. 17 and FIG. 18 represent flowcharts of an example of an operation of the monofocal type IOL measurement processing. Specifically, FIG. 17 represents a flowchart of an example of the operation of the monofocal type IOL measurement processing in step S5 of FIG. 16. FIG. 18 represents a flowchart of an example of the operation of the monofocal type IOL calculation processing in step S15 of FIG. 17.

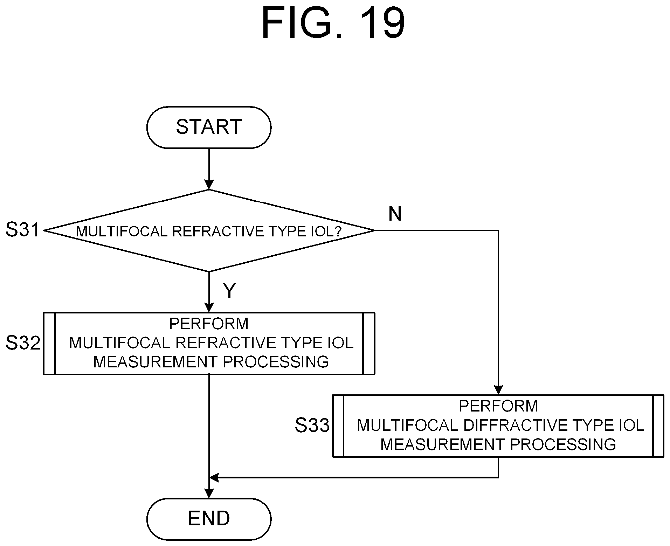

FIGS. 19 to 25 represent flowcharts of an example of an operation of the multifocal type IOL measurement processing. FIG. 19 represent a flowchart of an example of the operation of the multifocal type IOL measurement processing in step S7 of FIG. 16.

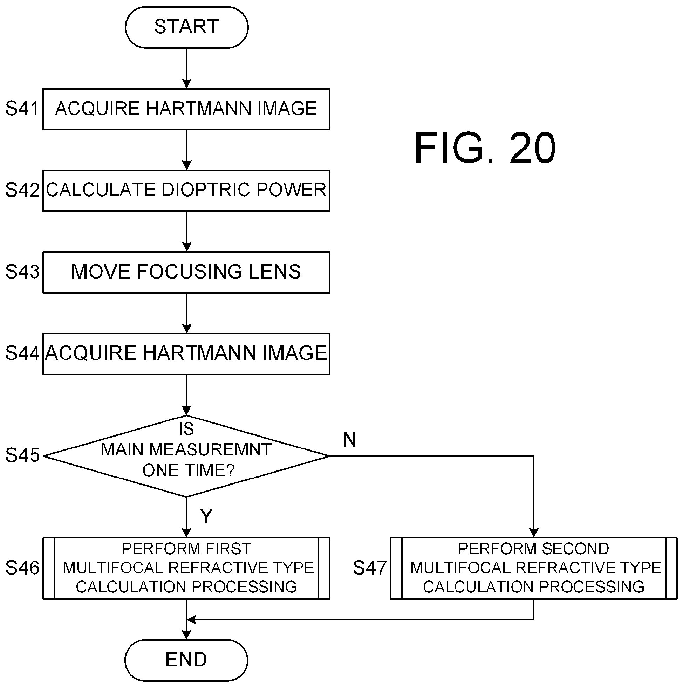

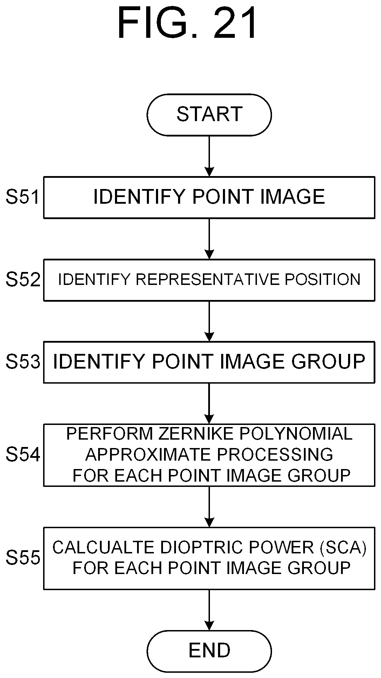

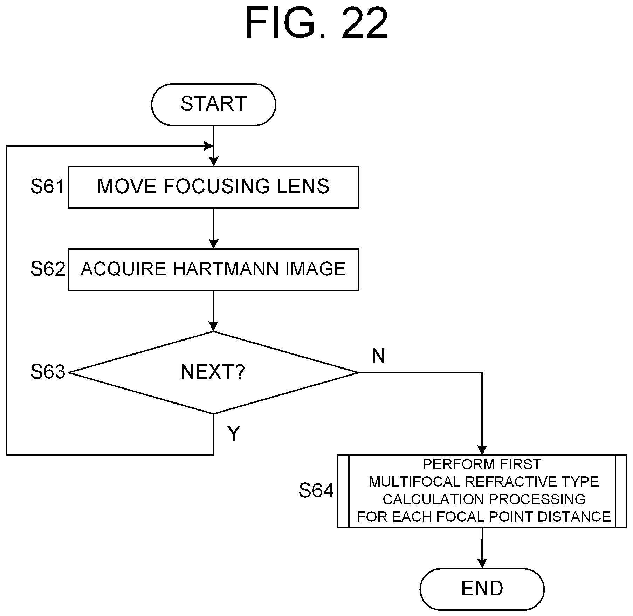

Specifically, FIGS. 20 to 22 represent flowcharts of an example of an operation of the multifocal refractive type IOL measurement processing. FIG. 20 represents a flowchart of an example of the operation of the multifocal refractive type IOL measurement processing in step S32 of FIG. 19. FIG. 21 represents a flowchart of an example of an operation of a first multifocal refractive type IOL calculation processing in step S46 of FIG. 20. FIG. 22 represents a flowchart of an example of an operation of a second multifocal refractive type IOL calculation processing in step S47 of FIG. 20.

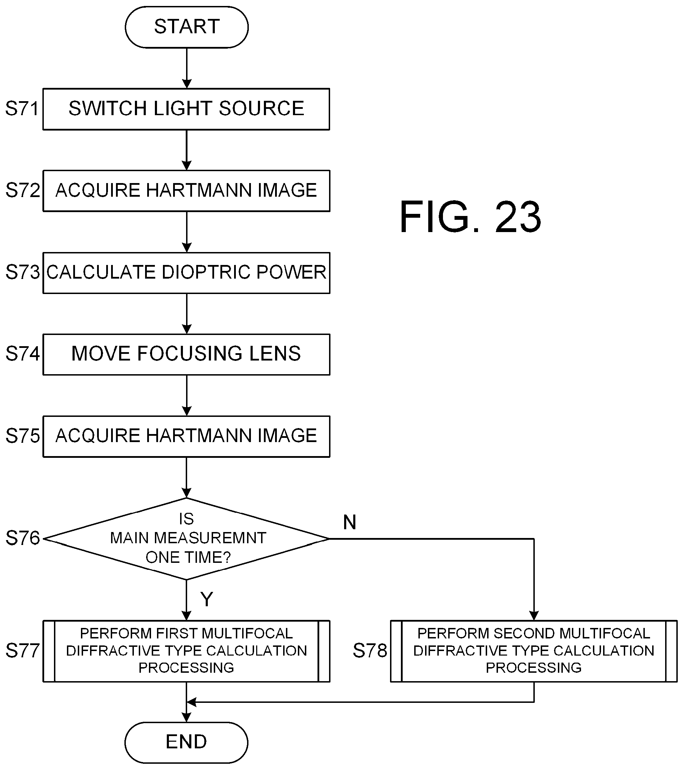

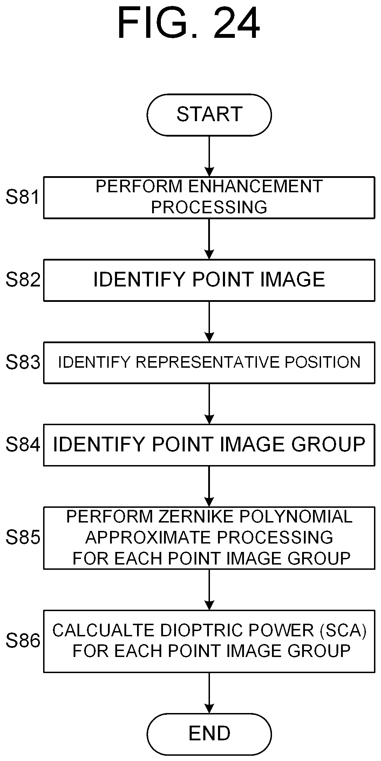

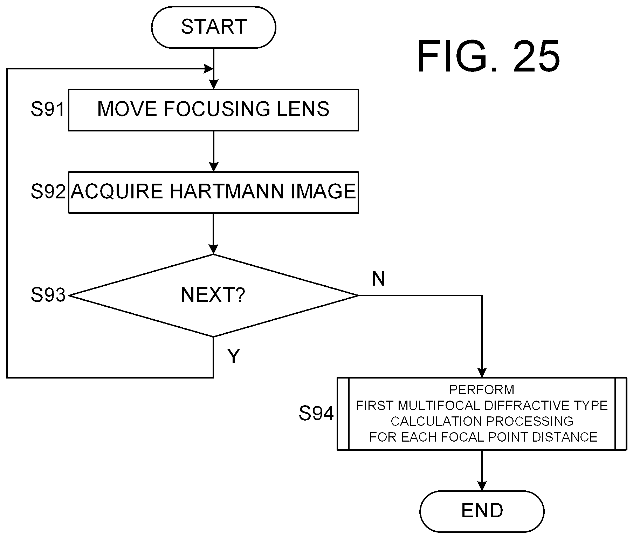

Further, FIGS. 23 to 25 represent flowcharts of an example of an operation of the multifocal diffractive type IOL measurement processing. Specifically, FIG. 23 represents a flowchart of an example of the operation of the multifocal diffractive type IOL measurement processing in step S33 of FIG. 19. FIG. 24 represents a flowchart of an example of an operation of a first multifocal diffractive type IOL calculation processing in step S77 of FIG. 23. FIG. 25 represents a flowchart of an example of an operation of a second multifocal diffractive type IOL calculation processing in step S78 of FIG. 23.

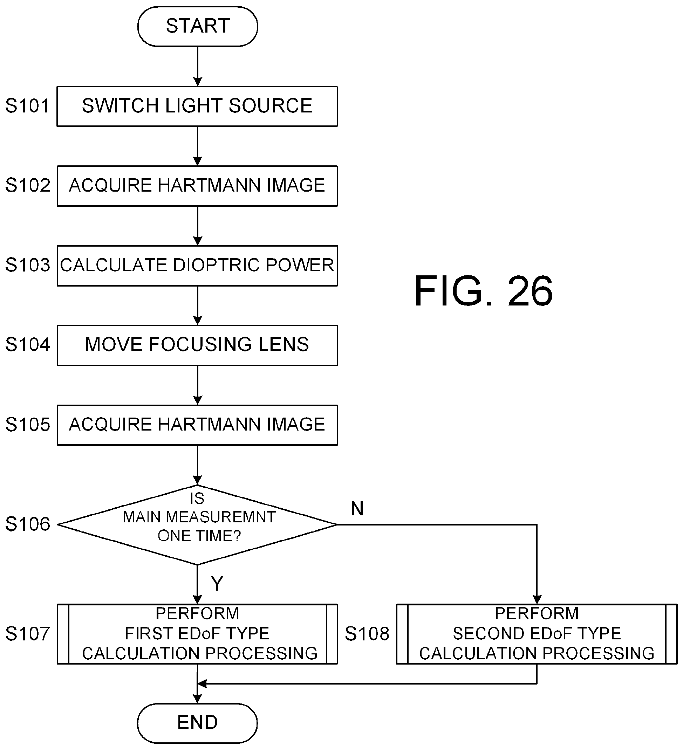

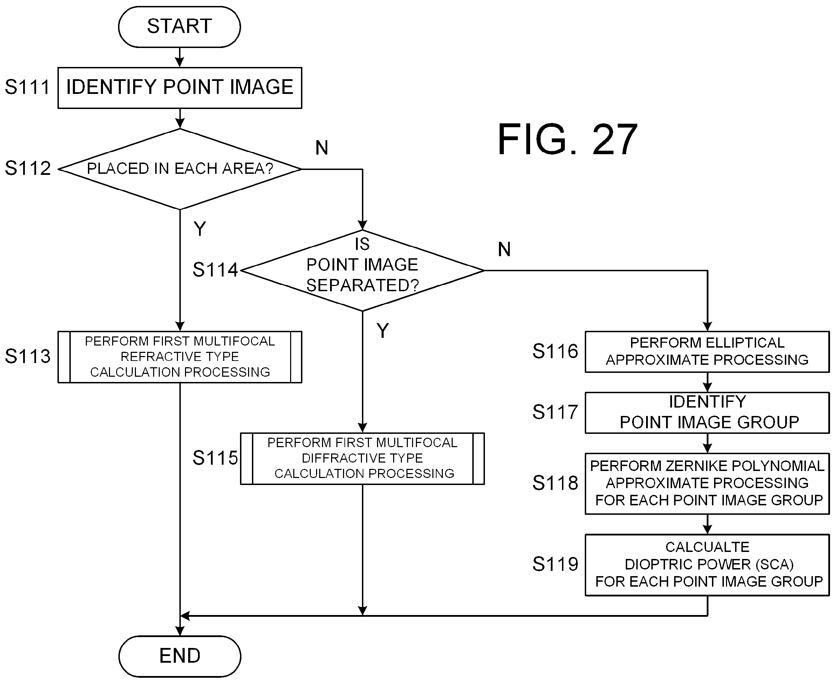

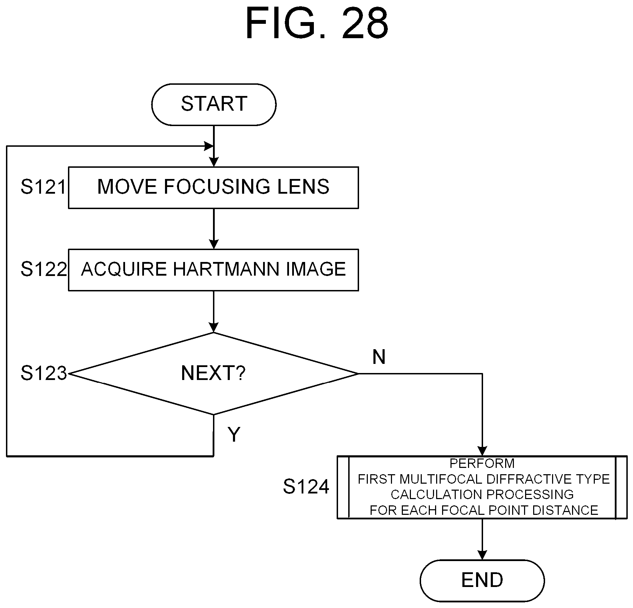

FIGS. 26 to 28 represent flowcharts of an example of an operation of the EDoF type IOL measurement processing. Specifically, FIG. 26 represents a flowchart of an example of the operation of the EDoF type IOL measurement processing in step S8 of FIG. 16. FIG. 27 represents a flowchart of an example of an operation of a first EDoF type IOL calculation processing in step S115 of FIG. 26. FIG. 28 represents a flowchart of an example of an operation of a second EDoF type IOL calculation processing in step S108 of FIG. 26.

The storage unit 112 stores computer programs for realizing the processing shown in FIGS. 16 to 28. The main controller 111 operates according to the computer programs, and thereby the main controller 111 performs the processing shown in FIGS. 16 to 28.

First, the flowchart of the example of the operation of the ophthalmic apparatus 100 shown in FIG. 16 will be described.

### (S1: Acquire IOL information)

First, the main controller 111 acquires IOL information worn by the eye E to be examined.

For example, the main controller 111 controls the communication unit 190 to acquire the IOL information representing the type of the IOL worn by the eye E to be examined from the electronic health record information of the examinee, that is stored in an external device such as an ophthalmic apparatus or server connected via the communication unit 190.

In some embodiments, the main controller 111 acquires the IOL information from the type of the IOL designated based on an operation content of a user to the operation unit 180.

In some embodiments, the main controller 111 controls the observation system 5 to acquire the transillumination image or the anterior segment image of the eye E to be examined after the completion of alignment described below, and controls the arithmetic processor 120 to analyze the transillumination image or the anterior segment image to determine the type of the IOL worn by the eye E to be examined and to acquire the determined type as the IOL information. In this case, for example, the main controller 111 can acquire the transillumination image by turning one of the anterior segment illumination light sources 57 on, illuminating the fundus Ef with the illumination light from a position away from the optical axis, and receiving the returning light of the light with the area sensor 56. Alternatively, for example, the main controller 111 acquires the transillumination image by switching the XY alignment light source 21 to the SLD or the high-brightness LED, projecting the light onto the fundus Ef, and receiving the returning light of the light with the area sensor 56. Further, the main controller 111 acquires the anterior segment image of the eye E to be examined by turning the anterior segment illumination light source 27 on, and receiving the returning light with the area sensor 56. The main controller 111 can determine the type of the IOL worn by the eye E to be examined and can acquire the IOL information, using a method disclosed in Japanese Unexamined Patent Application Publication No. 2014-209994, for example.

### (S2: Acquire pupil diameter information)

Subsequently, the main controller 111 acquires the pupil diameter information represents the pupil diameter of the eye E to be examined.

For example, the main controller 111 controls the communication unit 190 to acquire the pupil diameter information of the eye E to be examined from the electronic health record information of the examinee, that is stored in the external device such as an ophthalmic apparatus or server connected via the communication unit 190.

In some embodiments, the main controller 111 acquires the pupil diameter information from the pupil diameter designated based on an operation content of the user to the operation unit 180.

In some embodiments, the main controller 111 adjusts the brightness of the light source 41 and the optotype chart 42 in the optotype projection system 4 and controls the observation system 5 to acquire the anterior segment image of the eye E to be examined after the completion of alignment described below. And then, the main controller 111 controls the arithmetic processor 120 to analyze the anterior segment image to identify the pupil diameter of the eye E to be examined and to acquire the pupil diameter information. Here, the brightness of the light source 41 and the optotype chart 42 in the optotype projection system 4 can be set, for example, closer to the daily brightness of the eye E to be examined, set to the brightness of the state desired by the eye E to be examined, or set darker to allow analysis at any pupil diameter.

In some embodiments, the pupil diameter information representing the pupil diameter defined for a predetermined schematic eye is acquired as the pupil diameter information representing the pupil diameter of the eye E to be examined.

### (S3: Perform alignment)

Next, the examiner performs a predetermined operation on the operation unit 180 in a state where the face of the examinee is fixed to a face supporter (not shown), and then the ophthalmic apparatus 100 starts presenting the fixation target to the eye E to be examined. Specifically, the main controller 111 controls the optotype projection system 4 to present the fixation target to the eye E to be examined.

Subsequently, the examiner performs a predetermined operation on the operation unit 180 in a state where the face of the examinee is fixed to the face supporter, and then the ophthalmic apparatus 100 performs alignment. Thereby, the head unit is moved to an inspection position for the eye E to be examined through the XY alignment performed by using the XY alignment system 2 and the Z alignment performed by using the Z alignment system 1. The inspection position is a position where the inspection of the eye E to be examined can be performed within a default accuracy.

Specifically, the main controller 111 acquires imaging signal of the anterior segment image formed on the light receiving surface of the area sensor 56 and displays the anterior segment image E' on the display unit 170 (display screen 10a of the display unit 10). After that, the head unit is moved to the inspection position for the eye E to be examined through the XY alignment and the Z alignment described above. The movement of the head unit is executed by the main controller 111 in accordance with an instruction from the main controller 111. However, the movement of the head may be executed by the main controller 111 in accordance with an operation or an instruction by the user.

After the completion of alignment, the main controller 111 moves the movement unit 69 (light source 61), the movement unit 77, and the movement unit 46 to a position of the origin (for example, a position corresponding to 0D) along the optical axis, respectively. In some embodiments, the main controller 111 moves the movement unit 69 (light source 61), the movement unit 77, and the movement unit 46 to the position of the origin (for example, the position corresponding to 0D) along the optical axis, respectively, before the completion of alignment.

### (S4: Monofocal type IOL?)

Subsequently, the main controller 111 causes the measurement processing to be performed according to the type of the IOL worn by the eye E to be examined. The main controller 111 determines the type of the IOL worn by the eye E to be examined, based on the IOL information acquired in step S1. Here, the main controller 111 determines whether the IOL worn by the eye E to be examined is the monofocal type IOL, the multifocal type IOL, or the EDoF type IOL. In some embodiments, when it is determined that the IOL worn by the eye E to be examined is neither the monofocal type IOL, the multifocal type IOL, nor the EDoF type IOL, the main controller 111 displays information corresponding to a measurement error on the display unit 170 and stops the measurement processing of the dioptric power of the eye E to be examined.

In step S4, the main controller 111 determines whether or not the IOL worn by the eye E to be examined is the monofocal type IOL based on the IOL information acquired in step S1.

When it is determined in step S4 that the IOL worn by the eye E to be examined is the monofocal type IOL (S4: Y), the operation of the ophthalmic apparatus 100 proceeds to step S5. On the other hand, when it is determined that the IOL worn by the eye E to be examined is not the monofocal type IOL (S4: N), the operation of the ophthalmic apparatus 100 proceeds to step S6.

### (S5: Perform monofocal type IOL measurement processing)

When it is determined in step S4 that the IOL worn by the eye E to be examined is the monofocal type IOL (S4: Y), the main controller 111 causes the monofocal type IOL measurement processing to be performed. The details of step S5 will be described below. Subsequent to step S5, the operation of the ophthalmic apparatus 100 proceeds to step S9.

### (S6: Multifocal type IOL?)

When it is determined in step S4 that the IOL worn by the eye E to be examined is not the monofocal type IOL (S4: N), the main controller 111 determines whether or not the IOL worn by the eye E to be examined is the multifocal type IOL based on the IOL information acquired in step S1.

When it is determined in step S6 that the IOL worn by the eye E to be examined is the multifocal type IOL (S6: Y), the operation of the ophthalmic apparatus 100 proceeds to step S7. On the other hand, when it is determined that the IOL worn by the eye E to be examined is not the multifocal type IOL (S6: N), the operation of the ophthalmic apparatus 100 proceeds to step S8.

### (S7: Perform multifocal type IOL measurement processing)

When it is determined in step S6 that the IOL worn by the eye E to be examined is the multifocal type IOL (S6: Y), the main controller 111 causes the multifocal type IOL measurement processing to be performed. The details of step S7 will be described below. Subsequent to step S7, the operation of the ophthalmic apparatus 100 proceeds to step S9.

### (S8: Perform EDoF type IOL measurement processing)

When it is determined in step S6 that the IOL worn by the eye E to be examined is not the multifocal type IOL (S6: N), the main controller 111 causes the EDoF type IOL measurement processing to be performed. The details of step S8 will be described below. Subsequent to step S8, the operation of the ophthalmic apparatus 100 proceeds to step S9.

### (S9: Output)

Subsequent to step S5, step S7, or step S8, the main controller 111 displays the dioptric power calculated in step S5, step S7, or step S8 on the display unit 170. In some embodiments, the main controller 111 displays the wavefront aberration information, such as the wavefront aberration map, on the display unit 170 based on the measurement results obtained by performing the wavefront aberration measurement in association with the calculated dioptric power.

This terminates the operation of the ophthalmic apparatus 100 (END).

### [Monofocal type IOL measurement processing]

In step S5 in FIG. 16, the monofocal type IOL measurement processing is performed according to the flow shown in FIG. 17. In the case that the IOL worn by the eye E to be examined is the monofocal type IOL, the IOL has a single focal point distance. Therefore, a single dioptric power is calculated based on the point images that constitute the Hartmann image. In the monofocal type IOL measurement processing, after moving the focusing lens based on the dioptric power acquired by performing provisional measurement on the eye E to be examined, the dioptric power is calculated by performing the main measurement on the eye E to be examined. In other words, the focusing lens is moved to a position corresponding to the focal point distance of the IOL worn by the eye E to be examined, and the single dioptric power is calculated based on the acquired Hartmann image.

### (S11: Acquire Hartmann image)

First, the main controller 111 performs the provisional measurement, in step S5 in FIG. 16.

Specifically, the main controller 111 turns on the light source 61B to irradiate near-infrared light onto the eye E to be examined in a state where the optotype is presented to the eye E to be examined using the optotype chart 42, and causes the Hartmann image (point image groups) based on the returning light from the eye E to be examined to be detected by the area sensor 76.

### (S12: Calculate dioptric power)

Next, the main controller 111 controls the dioptric power calculator 130 to calculate the dioptric power based on the intervals between the point images constituting the Hartmann image detected by the area sensor 76, and identifies a movement amount of the movement unit 77 including the collimator lens 74 as the focusing lens (equivalent to the movement amount of the focusing lens) from the calculated dioptric power. In this case, the dioptric power calculator 130 calculates the spherical power S as the dioptric power, based on the intervals between the point images that make up the Hartmann image.

### (S13: Move focusing lens)

The main controller 111 moves the movement unit 77 (collimator lens 74 as the focusing lens) to a position corresponding to the far point along the optical axis based on the movement amount corresponding to the dioptric power (spherical power S) calculated in step S12. In conjunction with this, each of the movement unit 46 and the movement unit 69 also moves in the optical axis direction by the movement amount corresponding to the dioptric power described above.

In some embodiments, after the movement described above, the main controller 111 further determines whether or not this movement of the movement unit 77, etc. through the provisional measurement is a first movement. When it is determined that this movement is the first movement, the processing of step S5 in FIG. 16 proceeds to step S11 to perform the provisional measurement again. When it is determined that this movement is not the first movement, the processing of step S5 in FIG. 16 proceeds to step S14.

### (S14: Acquire Hartmann image)

Subsequently, the main controller 111 moves the movement unit 46 in the optical axis direction by another predetermined diopter from the position corresponding to the far point that has been moved in step S13, and causes the eye E to be examined to be promoted to the fogging of the optotype. In should be noted that the eye to be examined wearing the IOL is judged to have no amplitude of accommodation, the function for promoting to the fogging of the optotype may be omitted.

The main controller 111 turns on the light source 61B to irradiate near-infrared light onto the eye E to be examined, and causes the Hartmann image (point image groups) based on the returning light from the eye E to be examined to be detected by the area sensor 76, as the main measurement.

### (S15: Perform monofocal type calculation processing)

Sequentially, the main controller 111 controls the dioptric power calculator 130 to calculate the dioptric power based on the intervals between the point images constituting the Hartmann image detected by the area sensor 76. The dioptric power calculator 130 performs the monofocal type calculation processing to calculate the dioptric power of the eye E to be examined. The dioptric power calculated in step S15 includes the spherical power (S), the cylindrical power (astigmatic power) (C), and the astigmatic axis angle (A). The details of step S15 will be described below.

This terminates the processing of step S5 in FIG. 16 (END).

In step S15 in FIG. 17, the monofocal type IOL calculation processing is performed according to the flow shown in FIG. 18.

### (S21: Identify point image)

In step S15 of FIG. 17, first, the main controller 111 controls the point image identifying unit 131A in the first dioptric power calculator 131 to identify the point images constituting the Hartmann image acquired in step S14.

### (S22: Identify representative position)

Next, the main controller 111 controls the representative position identifying unit 131B to identify the representative position (in this case, position of the center of gravity) of the point image identified in step S21.

### (S23: Perform Zernike polynomial approximate processing)

Subsequently, the main controller 111 controls the Zernike polynomial approximate processor 131C to calculate the wavefront aberration information (approximate expression for wavefront) by performing Zernike polynomial approximate processing based on the representative position(s) of the plurality of point images identified in step S22 and the pupil diameter information acquired in step S2. Here, the wavefront aberration information is represented by the Zernike coefficient(s) and the Zernike polynomial. The Zernike polynomial approximate processor 131C normalizes the calculated wavefront aberration information using the pupil diameter information acquired in step S2.

### (S24: Calculate dioptric power (SCA))

Next, the main controller 111 controls the first dioptric power calculator 131 to obtain the spherical power S, the astigmatic power C, and the astigmatic axis angle A from the Zernike coefficients obtained by the Zernike polynomial approximate processing performed in step S23, using a known operation.

This terminates the processing of step S15 in FIG. 17 (END).

### [Multifocal type IOL measurement processing]

In step S7 of FIG. 16, the multifocal type IOL measurement processing is performed according to the flow shown in FIG. 19. In the multifocal type IOL measurement processing, the multifocal refractive type IOL measurement processing or the multifocal diffractive type IOL measurement processing is performed.

### [Multifocal refractive type IOL measurement processing]

In the case that the IOL worn by the eye E to be examined is the multifocal refractive type IOL, the IOL has a plurality of focal point distances. Therefore, a plurality of dioptric powers, each of which corresponds to each of the plurality of focal point distances, is calculated. In the multifocal refractive type IOL measurement processing, after moving the focusing lens to a position corresponding to an average dioptric power of the plurality of dioptric powers obtained by performing provisional measurement on the eye E to be examined, a main measurement is performed on the eye E to be examined, and the dioptric powers are calculated for each focal point distance. Alternatively, after moving the focusing lens to a position corresponding to each of the plurality of focal point distances, the dioptric power is calculated at each position. In other words, the focusing lens is moved to the position corresponding to the average focal point distance of the IOL worn by the eye E to be examined or the position corresponding to the focal point distance of the IOL, and the dioptric powers are calculated for each focal point distance of the IOL.

### (S31: Multifocal refractive type IOL?)

In step S7, the main controller 111 determines whether or not the IOL worn by the eye E to be examined is the multifocal refractive type IOL based on the IOL information acquired in step S1.

When it is determined in step S31 that the IOL worn by the eye E to be examined is the multifocal refractive type (S31: Y), the processing of step S7 in FIG. 16 proceeds to step S32. On the other hand, when it is determined that the IOL worn by the eye E to be examined is not the multifocal refractive type (S31: N), the processing of step S7 in FIG. 16 proceeds to step S33.

### (S32: Perform multifocal refractive type IOL measurement processing)

When it is determined in step S32 that the IOL worn by the eye E to be examined is the multifocal refractive type IOL (S32: Y), the main controller 111 causes the multifocal refractive type IOL measurement processing to be performed. The details of step S32 will be described below.

Subsequent to step S32, the processing of step S7 in FIG. 16 is terminated (END).

### (S33: Perform multifocal diffractive type IOL measurement processing)

When it is determined in step S32 that the IOL worn by the eye E to be examined is not the multifocal refractive type IOL (S32: N), the main controller 111 causes the multifocal diffractive type IOL measurement processing to be performed. The details of step S33 will be described below.

Subsequent to step S33, the processing of step S7 in FIG. 16 is terminated (END).

In step S32 of FIG. 19, the multifocal refractive type IOL measurement processing is performed according to the flow shown in FIG. 20.

### (S41: Acquire Hartmann image)

In step S32 of FIG. 19, first, the main controller 111 performs the provisional measurement in the same way as in step S11.

Specifically, the main controller 111 turns on the light source 61B to irradiate near-infrared light onto the eye E to be examined in a state where the optotype is presented to the eye E to be examined using the optotype chart 42, and causes the Hartmann image (point image groups) based on the returning light from the eye E to be examined to be detected by the area sensor 76.

### (S42: Calculate dioptric power)

Next, the main controller 111 controls the dioptric power calculator 130 to calculate the dioptric power based on the intervals between the point images constituting the Hartmann image detected by the area sensor 76, and identifies a movement amount of the movement unit 77 including the collimator lens 74 as the focusing lens (equivalent to the movement amount of the focusing lens) from the calculated dioptric power, in the same way as in step S12.

### (S43: Move focusing lens)

The main controller 111 moves the movement unit 77 (collimator lens 74 as the focusing lens) to a position corresponding to the far point along the optical axis based on the movement amount corresponding to the dioptric power (spherical power S) calculated in step S42, in the same way as in step S13. In conjunction with this, each of the movement unit 46 and the movement unit 69 also moves in the optical axis direction by the movement amount corresponding to the dioptric power described above.

### (S44: Acquire Hartmann image)

Subsequently, the main controller 111 moves the movement unit 46 in the optical axis direction by another predetermined diopter from the position corresponding to the far point that has been moved in step S33, and causes the eye E to be examined to be promoted to the fogging of the optotype, in the same way as in step S14. In should be noted that the eye to be examined wearing the IOL is judged to have no amplitude of accommodation, the function for promoting to the fogging of the optotype may be omitted.

The main controller 111 turns on the light source 61B to irradiate near-infrared light onto the eye E to be examined, and causes the Hartmann image (point image groups) based on the returning light from the eye E to be examined to be detected by the area sensor 76, as the main measurement.

### (S45: Is main measurement one time?)

Since the multifocal type IOL has a plurality of focal points, obtaining the dioptric power with only one main measurement may reduce the accuracy of the measurement. Therefore, the main controller 111 causes the main measurement to be performed a plurality of times in case that a predetermine condition is met. Examples of the predetermined condition include a first condition regarding a difference in dioptric power and a second condition regarding the number of point images, that can be identified, in the Hartmann image.

Examples of the first condition include "it is judged that the difference in the dioptric powers at the plurality of focal point distances of the IOL is large". When it is judged that the difference in the dioptric powers at the plurality of focal point distances of the IOL is large (in other words, when the first condition is met), the main controller 111 causes the main measurement to be performed a plurality of times. When it is judged that the difference in the dioptric powers at the plurality of focal point distances of the IOL is not large (in other words, when the first condition is not met), the main controller 111 causes the main measurement to be performed just one time.

For example, the main controller 111 controls the dioptric power calculator 130 to calculate the dioptric powers at the respective focal point distances in the provisional measurement and to calculate an average power of the plurality of calculated dioptric powers. The main controller 111 moves the movement unit 77 (collimator lens 74 as the focusing lens) to a position corresponding to the calculated average power along the optical axis. After then, the main controller 111 acquired the Hartmann image of the eye E to be examined, causes the dioptric power at each of the plurality of focal points to be calculated based on the acquired Hartmann image, and causes a power difference between the maximum dioptric power and the minimum dioptric power among the plurality of calculated dioptric powers to be calculated. When the calculated power difference is equal to or greater than a predetermined threshold, the main controller 111 causes the main measurement to be performed a plurality of times at each position in accordance with the focal point distance, and causes the dioptric power at each of the plurality of focal point distances to be calculated. Further, when the calculated power difference is less than the predetermined threshold, the main controller 111 causes the main measurement to be performed just one time to calculate the single dioptric power.

In the case where the number of the focal points of the IOL worn by the eye E to be examined is "2", for example, the main controller 111 causes the main measurement to be performed a plurality of times when the power difference is 4D (diopter) or more. And, the main controller 111 causes the main measurement to performed just one time, when the power difference is less than 4D.

Examples of the second condition include "it is judged that the number of the point images that can be identified from the Hartmann image is small". When it is judged that the number of the point images that can be identified from the Hartmann image is small (in other words, when the second condition is met), the main controller 111 causes the main measurement to be performed a plurality of times. When it is judged that the number of the point images that can be identified from the Hartmann image is not small (in other words, when the second condition is not met), the main controller 111 causes the main measurement to be performed just one time.

For example, the main controller 111 controls the dioptric power calculator 130 to calculate the dioptric powers at the respective focal point distances in the provisional measurement and to calculate an average power of the plurality of calculated dioptric powers. The main controller 111 moves the movement unit 77 (collimator lens 74 as the focusing lens) to a position corresponding to the calculated average power along the optical axis. After then, the main controller 111 acquires the Hartmann image of the eye E to be examined, and causes the point images in the acquired Hartmann image to be identified. When the number of the identified number of the point images is equal to or less than a predetermined threshold, the main controller 111 causes a plurality of main measurements to be performed at a position in accordance with the focal point distance, and causes the dioptric power at each of the plurality of focal point distances to be calculated. Further, when the number of the identified number of the point images is greater than the predetermined threshold, the main controller 111 causes the main measurements to be performed just one time, and causes the single dioptric power to be calculated.

In the case where the number of the focal points of the IOL worn by the eye E to be examined is "2", for example, the main controller 111 causes a plurality of main measurements to be performed when the number of the point images, that can be identified, is 50% or less of the total number of the point images within the pupil diameter. The main controller 111 causes the main measurement to performed just one time, when the number of the point images, that can be identified, is greater than 50% of the total number of the point images within the pupil diameter.

In step S45, the main controller 111 determines whether or not the main measurement is to be performed just one time, based on the first condition or the second condition described above. When it is determined in step S45 that the main measurement is to be performed just one time (S45: Y), the processing of step S32 in FIG. 19 proceeds to step S46. On the other hand, when it is determined in step S45 that the main measurement is to be performed a plurality of times (S45: N), the processing of step S32 in FIG. 19 proceeds to step S47.

### (S46: Perform first multifocal refractive type calculation processing)

When it is determined in step S45 that the main measurement is to be performed just one time (S45: Y), the main controller 111 causes the first multifocal refractive type calculation processing to be performed based on the intervals of the point images constituting the Hartmann image acquired in step S44 to calculate the dioptric power of the eye E to be examined. The dioptric power calculated in step S46 includes the spherical power (S), the cylindrical power (astigmatic power) (C), and the astigmatic axis angle (A). The details of step S46 will be described below. In step S46, the focusing lens is moved to a position corresponding to the dioptric power (average power of the plurality of focal point distances of the IOL) calculated based on the Hartmann image acquired by performing the provisional measurement (e.g., the Hartmann image acquired in step S44), and the dioptric power is calculated from the Hartmann image acquired at that position. Subsequent to step S46, the processing of step S32 in FIG. 19 is terminated (END).

### (S47: Perform second multifocal refractive type calculation processing)

When it is determined in step S45 that the main measurement is to be performed a plurality of times (S45: N), the main controller 111 causes the second multifocal refractive type calculation processing to be performed to calculate the dioptric power of the eye E to be examined. The dioptric power calculated in step S47 includes the spherical power (S), the cylindrical power (astigmatic power) (C), and the astigmatic axis angle (A). The details of step S47 will be described below. Subsequent to step S47, the processing of step S32 in FIG. 19 is terminated (END).

In step S46 of FIG. 20, the first multifocal refractive type calculation processing is performed according to the flow shown in FIG. 21.

### (S51: Identify point image)

In step S46 of FIG. 20, first, the main controller 111 controls the point image identifying unit 132A in the second dioptric power calculator 132 to identify the point images constituting the Hartmann image acquired in step S44. In some embodiments, the main controller 111 controls the second dioptric power calculator 132 to perform enhancement processing of point image on the Hartmann image acquired in step S44, and then controls the point image identifying unit 132A as described above.

### (S52: Identify representative position)

Next, the main controller 111 controls the representative position identifying unit 132B to identify the representative position (in this case, position of the center of gravity) of the point image identified in step S51.

### (S53: Identify point image group)

Subsequently, the main controller 111 controls the point image group identifying unit 132C to identify which area among a plurality of areas predetermined for each focal point distance of the IOL includes the point image constituting the Hartmann image based on the representative position identified in step S52. Here, if the position of the IOL within the pupil can be identified, the positional relationship indicating where each of the plurality of areas for each focal point distance of the IOL (areas for the number of focal points) are placed in the Hartmann image is known. Therefore, the point image group identifying unit 132C can identify in which area the representative position of the point images is placed based on the type of the IOL identified by the IOL information. The point image group identifying unit 132C classifies the point images by focal point distance (area) of the IOL (see FIG. 10 and FIG. 11). The point image group identifying unit 132C may identify the point image group by identifying an area where the intervals between the point images are narrowing with reference to a predetermined reference interval and an area where the intervals between the point images are widening with reference to a predetermined reference interval.

For example, when the number of the focal points of the IOL worn by the eye E to be examined is "2", the point image group identifying unit 132C identifies a point image group including one or more point images belonging to the near area(s) and a point image group including one or more point images belonging to the far area(s). For example, when the number of the focal points of the IOL worn by the eye E to be examined is "3", the point image group identifying unit 132C identifies a point image group including one or more point images belonging to the near area(s), a point image group including one or more point images belonging to one or more medium area(s), and a point image group including one or more point images belonging to the far area(s). For example, when the number of the focal points of the IOL worn by the eye E to be examined is 4 or more, the point image group identifying unit 132C identifies a point image group including one or more point images belonging to the near area(s), a point image group including one or more point images belonging to two or more medium area(s), and a point image group including one or more point images belonging to the far area(s).

### (S54: Perform Zernike polynomial approximate processing for each point image group)

Subsequently, the main controller 111 controls the Zernike polynomial approximate processor 132D to calculate the wavefront aberration information (approximate expression for wavefront), that is represented by the Zernike coefficient(s) and the Zernike polynomial, for each point image group by performing Zernike polynomial approximate processing for each point image group identified in step S53. The Zernike polynomial approximate processor 132D normalizes each of the calculated wavefront aberration information calculated for each point image group, using the pupil diameter information acquired in step S2.

### (S55: Calculate dioptric power (SCA) for each point image group)

Next, the main controller 111 controls the second dioptric power calculator 132 to obtain the spherical power S, the astigmatic power C, and the astigmatic axis angle A for each point image group from the Zernike coefficients obtained by the Zernike polynomial approximate processing performed for each point image group in step S54, using a known operation.

This terminates the first multifocal refractive type calculation processing of step S46 in FIG. 20 (END).

In step S47 of FIG. 20, the second multifocal refractive type calculation processing is performed according to the flow shown in FIG. 22.

### (S61: Move focusing lens)

In step S61 of FIG. 22, the main controller 111 moves the movement unit 77 (collimator lens 74 as the focusing lens) to a position corresponding to each of the plurality of focal point distances of the IOL along the optical axis, and sequentially causes the Hartmann image to be acquired at each position.

First, the main controller 111 moves the movement unit 77 (collimator lens 74 as the focusing lens) to a position corresponding to one of the plurality of focal point distances of the IOL along the optical axis. In conjunction with this, each of the movement unit 46 and the movement unit 69 also moves in the optical axis direction by the movement amount corresponding to the dioptric power described above.

### (S62: Acquire Hartmann image)

Subsequently, the main controller 111 moves the movement unit 46 in the optical axis direction by another predetermined diopter from the position corresponding to a position that has been moved in step S63, and causes the eye E to be examined to be promoted to the fogging of the optotype, in the same way as in step S14. In should be noted that the eye to be examined wearing the IOL is judged to have no amplitude of accommodation, the function for promoting to the fogging of the optotype may be omitted.

The main controller 111 turns on the light source 61B to irradiate near-infrared light onto the eye E to be examined, and causes the Hartmann image (point image groups) based on the returning light from the eye E to be examined to be detected by the area sensor 76, as the main measurement.

### (S63: Next?)

The main controller 111 causes the Hartmann image to be acquired at all of the plurality of focal point distances of the IOL. In the case where step S62 has not been performed for all of the plurality of focal point distances of the IOL, the main controller 111 determines that the focusing lens is to be moved to a next focal position. In the case where step S62 has been performed for all of the plurality of focal point distances of the IOL, the main controller 111 determines that the focusing lens is not to be moved to the next focal position.

When it is determined in step S63 that the focusing lens is to be moved to the next focal position (S63: Y), the processing of step S47 in FIG. 20 proceeds to step S61. On the other hand, when it is determined that the focusing lens is not to be moved to the next focal position (S63: N), the processing of step S47 in FIG. 20 proceeds to step S64.

### (S64: Perform first multifocal refractive type calculation processing for each focal point distance)

When it is determined in step S63 that the focusing lens is not to be moved to the next focal position (S63: N), the main controller 111 causes the first multifocal refractive type calculation processing to be performed for each focal point distance of the IOL, based on the Hartmann images sequentially acquired in step S62. In other words, the main controller 111 causes the first multifocal refractive type calculation processing shown in FIG. 21 to be performed for each focal point distance of the IOL, and causes the dioptric power of the eye E to be examined to be calculated for each focal point distance. The calculated dioptric power may be a dioptric power at a position corresponding to the respective focal point distance. However, a dioptric power of all groups may be calculated.

This terminates the second multifocal refractive type calculation processing of step S47 in FIG. 20 (END).

### [Multifocal diffractive type IOL measurement processing]

In the case where the IOL worn by the eye E to be examined is the multifocal diffractive type IOL, for example, the point images constituting the Hartmann image are separated, the separated point images are classified for each focal point distance of the IOL, and a plurality of dioptric powers are calculated for each focal point distance based on the classified two or more separated point images. In other words, the focusing lens is moved to a position corresponding to the average focal point distance of the IOL worn by the eye E to be examined or the position corresponding to the focal point distance of the IOL, and the dioptric power is calculated based on the two or more separated point images generated by separating the point images constituting the acquire Hartmann image. In the multifocal diffractive type IOL measurement processing, for example, the measurement wavelength is changed by switching the light source, and a plurality of dioptric powers of the eye E to be examined is calculated.

In step S33 of FIG. 19, the multifocal diffractive type IOL measurement processing is performed according to the flow shown in FIG. 23.

### (S71: Switch light source)

In step S33 of FIG. 19, first, the main controller 111 controls the light source 61 to switch the light source for measurement from the light source 61B to the light source 61A.

In some embodiments, in order to reduce the load of the analysis processing and to shorten the analysis time, the switching of the light source in step S71 is performed after performing the provisional measurement in step S75 or step 72. In this case, in the first try which is measured using near-infrared light, the point images at the far point alone are acquired. Or, even when the point images at the near point are also acquired, the point images at the far point alone can be easily selected since the intensity of the point image is weak. As a result, it becomes easier to identify and analyze the point images, and processing time can be reduced. In addition, it also reduces the burden on the human eye caused by visible light, and the miosis before the main measurement can be avoided.

### (S27: Acquire Hartmann image)

Subsequently, the main controller 111 causes the provisional measurement to be performed, in the same way as in step S11.

Specifically, the main controller 111 turns on the light source 61A to irradiate the visible light onto the eye E to be examined, and causes the Hartmann image (point image groups) based on the returning light from the eye E to be examined to be detected by the area sensor 76. Thereby, the wavefront aberration measurement can be performed using the visible light.

### (S73: Calculate dioptric power)

Next, the main controller 111 controls the dioptric power calculator 130 to calculate the dioptric power based on the intervals between the point images constituting the Hartmann image detected by the area sensor 76, and identifies a movement amount of the movement unit 77 including the collimator lens 74 as the focusing lens (equivalent to the movement amount of the focusing lens) from the calculated dioptric power, in the same way as in step S12. In the case where the separated point image are not acquired, the dioptric power is calculated from the acquired point image(s) to determine the movement amount. In the case where the point images is separated, the dioptric powers are calculated using each group of the points corresponding to the distant (for example, the points closer to the center of the area where the Hartmann image is depicted) and the points corresponding to the near objects (for example, the points further away from the center of the area where the Hartmann image is depicted) to determine the movement amount from the average value of the dioptric powers. Alternatively, the dioptric power may be calculated using solely the group of the points corresponding to the distant or solely the group of the points corresponding to the near object to determine the movement amount from the average of the dioptric powers.

### (S74: Move focusing lens)

The main controller 111 moves the movement unit 77 (collimator lens 74 as the focusing lens) to a position corresponding to the far point along the optical axis based on the movement amount corresponding to the dioptric power (spherical power S) calculated in step S73, in the same way as in step S13. In conjunction with this, each of the movement unit 46 and the movement unit 69 also moves in the optical axis direction by the movement amount corresponding to the dioptric power described above.

### (S75: Acquire Hartmann image)

Subsequently, the main controller 111 moves the movement unit 46 in the optical axis direction by another predetermined diopter from the position corresponding to the far point that has been moved in step S74, and causes the eye E to be examined to be promoted to the fogging of the optotype, in the same way as in step S14. In should be noted that the eye to be examined wearing the IOL is judged to have no amplitude of accommodation, the function for promoting to the fogging of the optotype may be omitted.

The main controller 111 turns on the light source 61A to irradiate near-infrared light onto the eye E to be examined, and causes the Hartmann image (point image groups) based on the returning light from the eye E to be examined to be detected by the area sensor 76, as the main measurement.

### (S76: Is main measurement one time?)

Subsequently, the main controller 111 determines whether or not the main measurement is to be performed just one time, based on the first condition or the second condition described above, in the same way as in step S45. When it is determined in step S76 that the main measurement is to be performed just one time (S76: Y), the processing of step S33 in FIG. 19 proceeds to step S77. On the other hand, when it is determined in step S76 that the main measurement is to be performed a plurality of times (S76: N), the processing of step S33 in FIG. 19 proceeds to step S78.

### (S77: Perform first multifocal diffractive type calculation processing)

When it is determined in step S76 that the main measurement is to be performed just one time (S76: Y), the main controller 111 causes the first multifocal diffractive type calculation processing to be performed based on the intervals of the point images constituting the Hartmann image acquired in step S75 to calculate the dioptric power of the eye E to be examined. The dioptric power calculated in step S46 includes the spherical power (S), the cylindrical power (astigmatic power) (C), and the astigmatic axis angle (A). The details of step S77 will be described below. In step S77, the focusing lens is moved to a position corresponding to the dioptric power (average power of the plurality of focal point distances of the IOL) calculated based on the Hartmann image acquired by performing the provisional measurement (e.g., the Hartmann image acquired in step S72 or step S75), and the dioptric power is calculated from the Hartmann image acquired at that position. Subsequent to step S77, the processing of step S33 in FIG. 19 is terminated (END).

### (S78: Perform second multifocal diffractive type calculation processing)

When it is determined in step S76 that the main measurement is to be performed a plurality of times (S76: N), the main controller 111 causes the second multifocal diffractive type calculation processing to be performed to calculate the dioptric power of the eye E to be examined. The dioptric power calculated in step S78 includes the spherical power (S), the cylindrical power (astigmatic power) (C), and the astigmatic axis angle (A). The details of step S78 will be described below. Subsequent to step S78, the processing of step S33 in FIG. 19 is terminated (END).

In step S77 of FIG. 23, the first multifocal refractive type calculation processing is performed according to the flow shown in FIG. 24.

### (S81: Perform enhancement processing)

In step S77 of FIG. 23, first, the main controller 111 controls the enhancement processor 133A in the third dioptric power calculator 133 to perform enhancement processing of the point image (separated point image) on the Hartmann image acquired in step S75. Thereby, the two or more separated point images (in particular, separated point image on the near point side), that are formed by separating the point image constituting the Hartmann image, can be easily identified.

### (S82: Identify point image)

Next, the main controller 111 controls the point image identifying unit 133B to identify the separated point image constituting the Hartmann image on which the enhancement processing of the separated point image has been performed in step S81.

### (S83: Identify representative position)

Next, the main controller 111 controls the representative position identifying unit 133C to identify the representative position (in this case, position of the center of gravity) of the separated point image identified in step S82.

### (S84: Identify point image group)

Subsequently, the main controller 111 controls the point image group identifying unit 133D to identify point image group for each focal point distance of the IOL, for the separated point image, based on the representative position identified in step S83. The point image group identifying unit 132C classifies the separated point images identified in step S82 into any one of the two or more point image groups determined according to the type of the IOL identified by the IOL information.

For example, when the number of the focal points of the IOL worn by the eye E to be examined is "2", the point image group identifying unit 133D identifies the point image group of the separated point images at the near point and the point image group of the separated point images at the far point, by classifying each of the plurality of separated point images identified in step S82 into either the point image group of the separated point images at the near point or the point image group of the separated point images at the far point. For example, when the number of the focal points of the IOL worn by the eye E to be examined is "3", the point image group identifying unit 133D identifies the point image group of the separated point images at the near point, the point image group of the separated point images at the medium point, and the point image group of the separated point images at the far point, by classifying each of the plurality of separated point images identified in step S82 into any one of the point image group of the separated point images at the near point, the point image group of the separated point images at the medium point, and the point image group of the separated point images at the far point. For example, when the number of the focal points of the IOL worn by the eye E to be examined is 4 or more, the point image group identifying unit 133D identifies the point image group of the separated point images at the near point, the two or more point image groups of the separated point images at the two or more medium points, and the point image group of the separated point images at the far point, by classifying each of the plurality of separated point images identified in step S82 into either the point image group of the separated point images at the near point, the two or more point image group of the separated point images at the two or more medium points, or the point image group of the separated point images at the far point.

### (S85: Perform Zernike polynomial approximate processing for each point image group)

Subsequently, the main controller 111 controls the Zernike polynomial approximate processor 133E to calculate the wavefront aberration information (approximate expression for wavefront), that is represented by the Zernike coefficient(s) and the Zernike polynomial, for each point image group by performing Zernike polynomial approximate processing for each point image group identified in step S84. The Zernike polynomial approximate processor 133E normalizes each of the calculated wavefront aberration information calculated for each point image group, using the pupil diameter information acquired in step S2.

### (S86: Calculate dioptric power (SCA) for each point image group)

Next, the main controller 111 controls the third dioptric power calculator 133 to obtain the spherical power S, the astigmatic power C, and the astigmatic axis angle A for each point image group from the Zernike coefficients obtained by the Zernike polynomial approximate processing performed for each point image group in step S85, using a known operation.

This terminates the first multifocal diffractive type calculation processing of step S77 in FIG. 23 (END).

In step S78 of FIG. 23, the second multifocal diffractive type calculation processing is performed according to the flow shown in FIG. 25.

### (S91: Move focusing lens)

In step S78 of FIG. 23, the main controller 111 moves the movement unit 77 (collimator lens 74 as the focusing lens) to a position corresponding to each of the plurality of focal point distances of the IOL along the optical axis, and sequentially causes the Hartmann image to be acquired at each position.

First, the main controller 111 moves the movement unit 77 (collimator lens 74 as the focusing lens) to a position corresponding to one of the plurality of focal point distances of the IOL along the optical axis. In conjunction with this, each of the movement unit 46 and the movement unit 69 also moves in the optical axis direction by the movement amount corresponding to the dioptric power described above.

### (S92: Acquire Hartmann image)

Subsequently, the main controller 111 moves the movement unit 46 in the optical axis direction by another predetermined diopter from the position corresponding to a position that has been moved in step S91, and causes the eye E to be examined to be promote to the fogging of the optotype, in the same way as in step S14. In should be noted that the eye to be examined wearing the IOL is judged to have no amplitude of accommodation, the function for promoting to the fogging of the optotype may be omitted.

The main controller 111 turns on the light source 61A to irradiate near-infrared light onto the eye E to be examined, and causes the Hartmann image (point image groups) based on the returning light from the eye E to be examined to be detected by the area sensor 76, as the main measurement.

### (S93: Next?)

The main controller 111 causes the Hartmann image to be acquired at all of the plurality of focal point distances of the IOL. In the case where step S92 has not been performed for all of the plurality of focal point distances of the IOL, the main controller 111 determines that the focusing lens is to be moved to a next focal position. In the case where step S92 has been performed for all of the plurality of focal point distances of the IOL, the main controller 111 determines that the focusing lens is not to be moved to the next focal position.

When it is determined in step S93 that the focusing lens is to be moved to the next focal position (S93: Y), the processing of step S78 in FIG. 23 proceeds to step S91. On the other hand, when it is determined that the focusing lens is not to be moved to the next focal position (S93: N), the processing of step S78 in FIG. 23 proceeds to step S94.

### (S94: Perform first multifocal diffractive type calculation processing for each focal point distance)

When it is determined in step S93 that the focusing lens is not to be moved to the next focal position (S93: N), the main controller 111 causes the first multifocal refractive type calculation processing to be performed for each focal point distance of the IOL, based on the Hartmann image sequentially acquired in step S92. In other words, the main controller 111 causes the first multifocal diffractive type calculation processing shown in FIG. 24 to be performed for each focal point distance of the IOL, and causes the dioptric power of the eye E to be examined to be calculated for each focal point distance. The calculated dioptric power may be a dioptric power at a position corresponding to the respective focal point distance. However, a dioptric power of all groups may be calculated.

This terminates the second multifocal diffractive type calculation processing of step S78 in FIG. 23 (END).

### [EDoF type IOL measurement processing]

In step S8 of FIG. 16, the EDoF type IOL measurement processing is performed according to the flow shown in FIG. 26. When the IOL worn by the eye E to be examined is the EDoF type IOL, the dioptric power of the eye E to be examined is calculated as a diffractive type IOL. In this case, for example, the two focal points of the approximate ellipse, which is identified by performing elliptical approximation on each of the plurality of point images that constitute the Hartmann image, are classified for each focal point distance, and the plurality of dioptric powers including the dioptric power corresponding to the far point and the dioptric power corresponding to the near point are calculated for each focal point distance based on the classified two or more separated point images. In other words, the focusing lens is moved to a position corresponding to the average focal point distance of the IOL worn by the eye E to be examined or the position corresponding to the focal point distance of the IOL, and the dioptric power is calculated based on the two focal points of the approximate ellipse identified by performing elliptical approximation on the point images constituting the acquire Hartmann image. In the EDoF type IOL measurement processing, for example , the measurement wavelength is changed by switching the light source, and a plurality of dioptric powers of the eye E to be examined are calculated.

In some embodiments, when the IOL worn by the eye E to be examined is the EDoF type IOL, the dioptric power of the eye E to be examined is calculated as the refractive type IOL. In this case, the dioptric power of the eye E to be examined is calculated in the same way as the multifocal refractive type IOL described above.

### (S101: Switch light source)

In step S8 of FIG. 16, first, the main controller 111 controls the light source 61 to switch the light source for measurement from the light source 61B to the light source 61A, in the same as in step S71. In some embodiments, in order to reduce the load of the analysis processing and to shorten the analysis time, the switching of the light source in step S71 is performed after performing the provisional measurement in step S105 or step 102. In this case, in the first try which is measured using near-infrared light, the point images at the far point alone are acquired. Or, even when the point images influenced by nearby objects are also acquired, the point images influenced by the distance alone can be acquired since the intensity of the point image is weak. As a result, it becomes easier to identify and analyze the point images, and processing time can be reduced. In addition, it also reduces the burden on the human eye caused by visible light, and the miosis before the main measurement can be avoided.

### (S102: Acquire Hartmann image)

Subsequently, the main controller 111 causes the provisional measurement to be performed, in the same way as in step S72.

Specifically, the main controller 111 turns on the light source 61A to irradiate the visible light onto the eye E to be examined, and causes the Hartmann image (point image groups) based on the returning light from the eye E to be examined to be detected by the area sensor 76.

### (S103: Calculate dioptric power)

Next, the main controller 111 controls the dioptric power calculator 130 to calculate the dioptric power based on the intervals between the point images constituting the Hartmann image detected by the area sensor 76, and identifies a movement amount of the movement unit 77 including the collimator lens 74 as the focusing lens (equivalent to the movement amount of the focusing lens) from the calculated dioptric power, in the same way as in step S73.

### (S104: Move focusing lens)

The main controller 111 moves the movement unit 77 (collimator lens 74 as the focusing lens) to a position corresponding to the far point along the optical axis based on the movement amount corresponding to the dioptric power (spherical power S) calculated in step S103, in the same way as in step S74. In conjunction with this, each of the movement unit 46 and the movement unit 69 also moves in the optical axis direction by the movement amount corresponding to the dioptric power described above.

### (S105: Acquire Hartmann image)

Subsequently, the main controller 111 moves the movement unit 46 in the optical axis direction by another predetermined diopter from the position corresponding to the far point that has been moved in step S104, and causes the eye E to be examined to be promoted to the fogging of the optotype, in the same way as in step S75. In should be noted that the eye to be examined wearing the IOL is judged to have no amplitude of accommodation, the function for promoting to the fogging of the optotype may be omitted.

The main controller 111 turns on the light source 61A to irradiate near-infrared light onto the eye E to be examined, and causes the Hartmann image (point image groups) based on the returning light from the eye E to be examined to be detected by the area sensor 76, as the main measurement.

### (S106: Is main measurement one time?)

Subsequently, the main controller 111 determines whether or not the main measurement is to be performed just one time, based on the first condition or the second condition described above, in the same way as in step S76. When it is determined in step S106 that the main measurement is to be performed just one time (S106: Y), the processing of step S8 in FIG. 16 proceeds to step S107. On the other hand, when it is determined in step S106 that the main measurement is to be performed a plurality of times (S106: N), the processing of step S8 in FIG. 16 proceeds to step S108.

### (S107: Perform first EDoF type calculation processing)

When it is determined in step S106 that the main measurement is to be performed just one time (S106: Y), the main controller 111 causes the first EDoF type calculation processing to be performed based on the intervals of the point images constituting the Hartmann image acquired in step S105 to calculate the dioptric power of the eye E to be examined. The dioptric power calculated in step S107 includes the spherical power (S), the cylindrical power (astigmatic power) (C), and the astigmatic axis angle (A). The details of step S107 will be described below. In step S107, the focusing lens is moved to a position corresponding to the dioptric power (average power of the plurality of focal point distances of the IOL) calculated based on the Hartmann image acquired by performing the provisional measurement (e.g., the Hartmann image acquired in step S102 or step S105), and the dioptric power is calculated from the Hartmann image acquired at that position. Subsequent to step S107, the processing of step S8 in FIG. 16 is terminated (END).

### (S108: Perform second EDoF type calculation processing)

When it is determined in step S106 that the main measurement is to be performed a plurality of times (S106: N), the main controller 111 causes the second EDoF type calculation processing to be performed to calculate the dioptric power of the eye E to be examined. The dioptric power calculated in step S108 includes the spherical power (S), the cylindrical power (astigmatic power) (C), and the astigmatic axis angle (A). The details of step S108 will be described below. Subsequent to step S108, the processing of step S8 in FIG. 16 is terminated (END).

In step S107 of FIG. 26, the first EDoF type IOL calculation processing is performed according to the flow shown in FIG. 27.

### (S111: Identify point image)

In step S107 in FIG. 26, first, the main controller 111 controls the point image identifying unit 134A in the fourth dioptric power calculator 134 to identify the point image constituting the Hartmann image acquired in step S105. In some embodiments, the main controller 111 controls the fourth dioptric power calculator 134 to perform enhancement processing of point image on the Hartmann image acquired in step S105, and then controls the point image identifying unit 134A as described above.

### (S112: Placed in each area?)

Next, the main controller 111 determines whether or not the plurality of the point images identified in step S111 is placed each area corresponding to the focal point distance of the IOL, as shown in FIG. 10 or FIG. 11. In some embodiments, the main controller 111 controls the fourth dioptric power calculator 134 to identify the representative position of the point image identified in step S111 in the same way as in step S52, and determines whether or not the representative position is placed in each area corresponding to the focal point distance of the IOL, based on the identified representative position in the same way as in step S53.

When it is determined that the plurality of identified point images is placed in each area corresponding to the focal point distance of the IOL (S112: Y), the processing of step S107 in FIG. 26 proceeds to step S113. When it is determined that the plurality of identified point images is not placed in each area corresponding to the focal point distance of the IOL (S112: N), the processing of step S107 in FIG. 26 proceeds to step S114.

### (S113: Perform first multifocal refractive type calculation processing)

When it is determined in step S112 that the plurality of identified point images is placed in each area corresponding to the focal point distance of the IOL (S112: Y), the main controller 111 controls the second dioptric power calculator 132 to perform the first multifocal refractive type calculation processing, in which the dioptric power of the eye E to be examined is calculated, based on the point image(s) identified in step S111. The second dioptric power calculator 132 performs the first multifocal refractive type calculation processing according to the flow shown in FIG. 21. This terminates the processing of step S107 in FIG. 26 (END).

### (S114: Is point image separated?)

When it is determined in step S112 that the plurality of identified point images are not placed in each area corresponding to the focal point distance of the IOL (S112: N), the main controller 111 determines whether or not the point images, the number of which is equal to or greater than a predetermined threshold value among the point images constituting the Hartmann image, are separated into the two or more separated point images as shown in FIG. 13, for the point images identified in step S111.

When it is determined that the point images, the number of which is equal to or greater than a predetermined threshold value among the identified point images, are separated into the two or more separated point images (S114: Y), the processing of step S107 in FIG. 26 proceeds to step S115. When it is determined that the point images, the number of which is equal to or greater than a predetermined threshold value among the identified point images, are not separated into the two or more separated point images (S114: N), the processing of step S107 in FIG. 26 proceeds to step S116.

### (S115: Perform first multifocal diffractive type calculation processing)

When it is determined that the point images, the number of which is equal to or greater than a predetermined threshold value among the identified point images, are separated into the two or more separated point images (S114: Y), the main controller 111 controls the third dioptric power calculator 133 to perform the first multifocal diffractive type calculation processing in which the dioptric power of the eye E to be examined is calculated based on the point image(s) identified in step S111. The third dioptric power calculator 133 performs the first multifocal diffractive type calculation processing according to the flow shown in FIG. 24. This terminates the processing of step S107 in FIG. 26 (END).

### (S116: Perform elliptical approximate processing)

When it is determined in step S114 that the point images, the number of which is equal to or greater than a predetermined threshold value among the identified point images, are not separated into the two or more separated point images (S114: N), the main controller 111 controls the elliptical approximate processor 134B to perform known elliptical approximate processing on each of the point images identified in step S111. The elliptical approximate processor 134B identifies a plurality of approximate ellipses of the point images identified in step S111, and identifies the two focal points of each of the identified approximate ellipses.

### (S117: Identify point image group)

Next, the main controller 111 controls the point image group identifying unit 134C to identify the focal point closer to the center of the Hartmann image among the two focal points of the approximate ellipse identified in step S116 as the focal point at near point, and the focal point farther from the center of the Hartmann image among the two focal points of the approximate ellipse identified in step S116 as the focal point at far point. The point image group identifying unit 134C classifies the plurality of point images at near point and the plurality of point images at far point, which are identified for each of the plurality of approximate ellipses, into a point image group of the point image at near point and a point image group of the point image at far point.

### (S118: Perform Zernike polynomial approximate processing for each point image group)

Subsequently, the main controller 111 controls the Zernike polynomial approximate processor 134D to calculate the wavefront aberration information (approximate expression for wavefront), that is represented by the Zernike coefficient(s) and the Zernike polynomial, for each point image group by performing Zernike polynomial approximate processing for each point image group identified in step S117. The Zernike polynomial approximate processor 134D normalizes each of the calculated wavefront aberration information calculated for each point image group, using the pupil diameter information acquired in step S2.

### (S119: Calculate dioptric power (SCA) for each point image group)

Next, the main controller 111 controls the fourth dioptric power calculator 134 to obtain the spherical power S, the astigmatic power C, and the astigmatic axis angle A for each point image group from the Zernike coefficients obtained by the Zernike polynomial approximate processing performed for each point image group in step S118, using a known operation.

This terminates the processing of step S107 in FIG. 26 (END).

In step S108 of FIG. 26, the second EDoF type IOL calculation processing is performed according to the flow shown in FIG. 28.

### (S121: Move focusing lens)

In step S108 of FIG. 26, the main controller 111 moves the movement unit 77 (collimator lens 74 as the focusing lens) to a position corresponding to each of the plurality of focal point distances of the IOL along the optical axis, and sequentially causes the Hartmann image to be acquired at each position.

First, the main controller 111 moves the movement unit 77 (collimator lens 74 as the focusing lens) to a position corresponding to one of the plurality of focal point distances of the IOL along the optical axis. In conjunction with this, each of the movement unit 46 and the movement unit 69 also moves in the optical axis direction by the movement amount corresponding to the dioptric power described above.

### (S122: Acquire Hartmann image)

Subsequently, the main controller 111 moves the movement unit 46 in the optical axis direction by another predetermined diopter from the position corresponding to a position that has been moved in step S111, and causes the eye E to be examined to be promote to the fogging of the optotype, in the same way as in step S92. In should be noted that the eye to be examined wearing the IOL is judged to have no amplitude of accommodation, the function for promoting to the fogging of the optotype may be omitted.

The main controller 111 turns on the light source 61A to irradiate near-infrared light onto the eye E to be examined, and causes the Hartmann image (point image groups) based on the returning light from the eye E to be examined to be detected by the area sensor 76, as the main measurement.

### (S123: Next?)

The main controller 111 causes the Hartmann image to be acquired at all of the plurality of focal point distances of the IOL. In the case where step S122 has not been performed for all of the plurality of focal point distances of the IOL, the main controller 111 determines that the focusing lens is to be moved to a next focal position. In the case where step S122 has been performed for all of the plurality of focal point distances of the IOL, the main controller 111 determines that the focusing lens is not to be moved to the next focal position.

When it is determined in step S123 that the focusing lens is to be moved to the next focal position (S123: Y), the processing of step S108 in FIG. 26 proceeds to step S121. On the other hand, when it is determined that the focusing lens is not to be moved to the next focal position (S123: N), the processing of step S108 in FIG. 26 proceeds to step S124.

### (S124: Perform first multifocal diffractive type calculation processing for each focal point distance)

When it is determined in step S123 that the focusing lens is not to be moved to the next focal position (S123: N), the main controller 111 causes the first multifocal diffractive type calculation processing to be performed for each focal point distance of the IOL, based on the Hartmann image sequentially acquired in step S122. In other words, the main controller 111 causes the first multifocal diffractive type calculation processing shown in FIG. 24 to be performed for each focal point distance of the IOL, and causes the dioptric power of the eye E to be examined to be calculated for each focal point distance. The calculated dioptric power may be a dioptric power at a position corresponding to the respective focal point distance. However, a dioptric power of all groups may be calculated.

This terminates the second EDoF type calculation processing of step S108 in FIG. 26 (END).

As described above, according to the embodiments, the measurement processing method (including the calculation method of dioptric power) is changed according to the type of the IOL worn by the eye E to be examined. Thereby, the reliability of the calculation result of the dioptric power of the eye to be examined wearing an IOL can be improved.

### (Modification examples)

The processing according to the embodiments is not limited to the processing described in the above embodiments. For example, step S77 or step S78 in FIG. 23 may be performed as shown below.

### <First modification example>

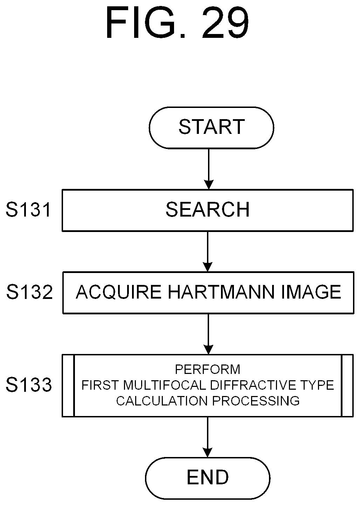

FIG. 29 shows an example of a processing flow of step S77 according to a first modification example of the embodiments.

The storage unit 112 stores a computer program for realizing the processing shown in FIG. 29. The main controller 111 operates according to the computer program, and thereby the main controller 111 performs the processing shown in FIG. 29.

### (S131: Search)

As described above, in the case where the IOL worn by the eye E to be examined is the multifocal diffractive type IOL, the point image constituting the Hartmann image is separated into two or more separated point images. In this case, the contour of the separated point image on the near side is often not clear. Therefore, the main controller 111 controls each part to search for a position where the separated point image (on the near side) becomes clear while moving the focusing lens.

The main controller 111 repeats the acquisition of the Hartmann image by controlling the aberration measurement projection system 6 and the aberration measurement light reception system 7, while moving the movement unit 77 by a predetermined step toward the near point side from a position corresponding to the far point that has been moved in step S74. For example, the main controller 111 controls the dioptric power calculator 130 (arithmetic processor 120) to perform enhancement processing on each of the Hartmann images that have been repeatedly acquired. Alternatively, the main controller 111 may control the aberration measurement projection system 6 and aberration measurement light reception system 7 so as to repeatedly acquire the Hartmann image, by controlling the light source 61A so as to increase the light intensity, or by increasing the gain of the area sensor 76 or lengthening the exposure time.

The main controller 111 controls the dioptric power calculator 130 (arithmetic processor 120) as a search processor to search for a position where the separated point image can be successfully identified (position of the focusing lens (movement unit 77)) by analyzing the acquired Hartmann image. In some embodiments, the dioptric power calculator 130 (arithmetic processor 120) identifies a position where the contrast of the lowest contrast separated point image among the two or more separated point images separated from a single point image is highest as the position where the separated point image can be successfully identified. In some embodiments, the dioptric power calculator 130 (arithmetic processor 120) identifies a position where the interval between the two or more separated point images (interval between the representative positions) is widest as the position where the separated point image can be successfully identified. In some embodiments, the dioptric power calculator 130 (arithmetic processor 120) identifies a position where the number of the identified separated point images is the largest as the position where the separation point images can be successfully identified.

### (S132: Acquire Hartmann image)

Subsequently, the main controller 111 turns on the light source 61A to irradiate the near-infrared light onto the eye E to be examined, and causes the Hartmann image (point image groups) based on the returning light from the eye E to be examined to be detected by the area sensor 76, as the main measurement.

### (S133: Perform first multifocal diffractive type calculation processing)

The main controller 111 causes the first multifocal diffractive type calculation processing to be performed based on the intervals of the point images constituting the Hartmann image acquired in step S132 to calculate the dioptric power of the eye E to be examined. The dioptric power calculated in step S137 includes the spherical power (S), the cylindrical power (astigmatic power) (C), and the astigmatic axis angle (A). Subsequent to step S133, the processing of step S77 in FIG. 23 is terminated (END). It should be noted that in the case where the number of point images to be separated is large, the positions, each of which becomes clear, may be searched for, and the dioptric powers, each of which corresponding to each of the positions, may be calculated as a result.

### <Second modification example>

Generally, it is thought that the eye wearing the IOL will lose the function of accommodation. However, there is a possibility that the function of accommodation can be restored to the eye to be examined wearing the IOL through the movement of the ciliary muscle, etc. Therefore, in the second modification example of the embodiments, the wavefront aberration measurement is performed at two or more distances including the far point side and the near point side, and a distant power (dioptric power at far point) and a near (reading) power (dioptric power at near point) are calculated at each position.

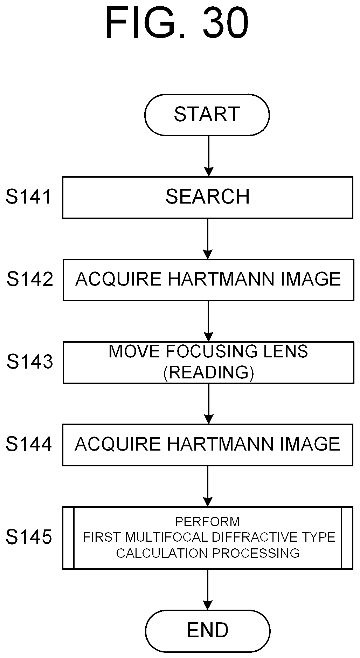

FIG. 30 shows an example of a processing flow of step S78 according to a second modification example of the embodiments.

The storage unit 112 stores a computer program for realizing the processing shown in FIG. 30. The main controller 111 operates according to the computer program, and thereby the main controller 111 performs the processing shown in FIG. 30.

### (S141: Search)

In step S74 of FIG. 23, which is performed prior to step S141, the movement unit 77 ( collimator lens 74 as the focusing lens) is moved to a position corresponding to the far point along the optical axis based on the movement amount corresponding to the dioptric power (spherical power S) calculated in step S73.

In step S141, the main controller 111 controls each part to search for a position where the separated point image becomes clear while moving the focusing lens, in the same way as in step S131.

### (S142: Acquire Hartmann image)

The main controller 111 moves the movement unit 46 in the optical axis direction by another predetermined diopter from the position (distant position) corresponding to the far point that has been moved in step S74, and causes the eye E to be examined to be promoted to the fogging of the optotype. In should be noted that the eye to be examined wearing the IOL is judged to have no amplitude of accommodation, the function for promoting to the fogging of the optotype may be omitted.

Subsequently, the main controller 111 moves the movement unit 77 to the position that has been searched for in step S141, and turns on the light source 61A to irradiate the visible light onto the eye E to be examined, and causes the Hartmann image (point image groups) based on the returning light from the eye E to be examined to be detected by the area sensor 76, as the main measurement, in the same way as in step S132.

### (S143: Move focusing lens (reading))

The main controller 111 moves the movement unit 77 (collimator lens 74 as the focusing lens) to a reading position (near vision position) along the optical axis. In conjunction with this, each of the movement unit 46 and the movement unit 69 also moves in the optical axis direction by the movement amount corresponding to the dioptric power described above. Examples of the reading position include a fixed position such as 40 cm, a position frequently used by the eye E to be examined, and a predetermined reading position corresponding to the IOL worn by the eye E to be examined.

### (S144: Acquire Hartmann image)

Next, the main controller 111 irradiates the light (near-infrared light or visible light) from the light source 61 (light source 61A or light source 61B) onto the eye E to be examined, and causes the Hartmann image (point image groups) based on the returning light from the eye E to be examined to be detected by the area sensor 76, as the main measurement.

### (S145: Perform first multifocal diffractive type calculation processing)

The main controller 111 causes the first multifocal diffractive type calculation processing to be performed based on the intervals of the point images constituting the Hartmann image detected by the area sensor 76 in step S75 or step S144 to calculate the dioptric power of the eye E to be examined. The dioptric power calculated in step S145 includes the spherical power (S), the cylindrical power (astigmatic power) (C), and the astigmatic axis angle (A). Subsequent to step S145, the processing of step S78 in FIG. 23 is terminated (END).

For example, the distant power and the reading power can be obtained from the Hartmann image acquired by performing distant measurement, and the distant power and the reading power can be obtained from the Hartmann image acquired by performing reading measurement. In this case, the ophthalmic apparatus may select and output the distant power acquired by performing the distant measurement and the reading power acquired by performing the reading measurement.

As described above, according to the modification examples, the plurality of Hartmann images, each of which is acquired in a state of being focused on the eye E to be examined corresponding to each of the plurality of focal point distances of the IOL, and the dioptric power is calculated using each of the Hartmann images. Thereby, even when the eye E to be examined wearing the IOL has the function of accommodation, the reliability of the calculation results of the dioptric power of the eye E to be examined wearing the IOL can be improved.

Alternatively, as another modification example of the embodiments, when the information on the reading power of the IOL is known, the dioptric power at the distance may be calculated, then the focusing lens may be moved by the amount of the additional power of the design value, and the Hartmann image may be acquired at that position to analyze the acquired Hartmann image. Alternatively, for example, the Hartmann image may also be acquired and be analyzed, while sequentially moving the focusing lens by a predetermined number of powers, such as from +5D to -5D by -1D.

### [Actions]

The ophthalmic apparatus, the method of controlling the ophthalmic apparatus, and the program according to the embodiments will be explained.

The first aspect of the embodiments is an ophthalmic apparatus (100) including a measurement optical system (aberration measurement projection system 6 and aberration measurement light reception system 7), an acquisition unit (communication unit 190), a controller (110, main controller 111), and a calculator (dioptric power calculator 130). The measurement optical system includes a focusing lens (collimator lens 74), and is configured to measure wavefront aberration of an eye (E) to be examined wearing an intraocular lens to acquire a Hartmann image. The acquisition unit is configured to acquire intraocular lens information representing at least an optical characteristics of the intraocular lens. The controller is configured to move the focusing lens to a position corresponding to a focal point distance of the intraocular lens determined based on the intraocular lens information and to cause the measurement optical system to acquire the Hartmann image. The calculator is configured to calculate a dioptric power of the eye to be examined based on the Hartmann image using an arithmetic processing method corresponding to the intraocular lens information.

According to such an aspect, the dioptric power of the eye to be examined wearing the IOL is calculated using a measurement method in accordance with the type of the IOL worn by the eye to be examined. Thereby, the reliability of the calculation result of the dioptric power of the eye to be examined wearing an IOL can be improved.

In the second aspect of the embodiments, in the first aspect, the acquisition unit is configured to acquire pupil diameter information representing a pupil diameter, and the calculator is configured to calculate the dioptric power based on the Hartmann image within a region demarcated based on the pupil diameter information.

According to such an aspect, the dioptric power is calculated based on the wavefront aberration information corresponding to the pupil diameter. Thereby, the reliability of the calculation result of the dioptric power of the eye to be examined wearing an IOL can be further improved.

In the third aspect of the embodiments, in the first aspect or the second aspect, the intraocular lens information represents either a monofocal type or a multifocal type.

According to such an aspect, the dioptric power of the eye to be examined wearing the **IOL** is calculated using a measurement method in accordance with either the monofocal type **IOL or** the multifocal type **IOL.** Thereby, the reliability of the calculation result of the dioptric power of the eye to be examined wearing an **IOL** can be improved.

**In** the fourth aspect of the embodiments, in the third aspect, when the intraocular lens is determined to be a monofocal type intraocular lens based on the intraocular lens information, the controller is configured to move the focusing lens to a position corresponding to a focal point distance of the intraocular lens and the calculator is configured to calculate a single dioptric power based on the Hartmann image.

According to such an aspect, the reliability of the calculation result of the dioptric power of the eye to be examined wearing the monofocal type **IOL** can be improved.

**In** the fifth aspect of the embodiments, in the third aspect or the fourth aspect, when the intraocular lens is determined to be a multifocal refractive type intraocular lens based on the intraocular lens information, the controller is configured to move the focusing lens to a position corresponding to an average focal point distance of the intraocular lens or a position corresponding to a focal point distance of the intraocular lens and the calculator is configured to calculate the dioptric power for each focal point distance of the intraocular lens.

According to such an aspect, the reliability of the calculation result of the dioptric power of the eye to be examined wearing the multifocal refractive type IOL can be improved.

In the sixth aspect of the embodiments, in any one of the third aspect to the fifth aspect, when the intraocular lens is determined to be a multifocal diffractive type intraocular lens based on the intraocular lens information, the controller is configured to move the focusing lens to a position corresponding to an average focal point distance of the intraocular lens or a position corresponding to a focal point distance of the intraocular lens and the calculator is configured to calculate the dioptric power based on two or more separated point images that are separated from point images that make up the Hartmann image.

According to such an aspect, the reliability of the calculation result of the dioptric power of the eye to be examined wearing the multifocal diffractive type IOL can be improved.

In the seventh aspect of the embodiments, in any one of the third aspect to the sixth aspect, when the intraocular lens is determined to be an extended depth of focus type intraocular lens based on the intraocular lens information, the controller is configured to move the focusing lens to a position corresponding to an average focal point distance of the intraocular lens or a position corresponding to a focal point distance of the intraocular lens and the calculator is configured to calculate the dioptric power based on two focal points of an approximate ellipse identified by performing elliptical approximation on point images that make up the Hartmann image.

According to such an aspect, the reliability of the calculation result of the dioptric power of the eye to be examined wearing the EDoF type IOL can be improved.

The eighth aspect of the embodiments is a method of controlling an ophthalmic apparatus (100) including a measurement optical system (aberration measurement projection system 6 and aberration measurement light reception system 7) including a focusing lens (collimator lens 74) and configured to measure wavefront aberration of an eye (E) to be examined wearing an intraocular lens to acquire a Hartmann image. The method of controlling the ophthalmic apparatus includes: an acquisition step of acquiring intraocular lens information representing at least an optical characteristics of the intraocular lens; a control step of moving the focusing lens to a position corresponding to a focal point distance of the intraocular lens determined based on the intraocular lens information and of causing the measurement optical system to acquire the Hartmann image; and a calculation step of calculating a dioptric power of the eye to be examined based on the Hartmann image using an arithmetic processing method corresponding to the intraocular lens information.

According to such a method, the dioptric power of the eye to be examined wearing the IOL is calculated using a measurement method in accordance with the type of the IOL worn by the eye to be examined. Thereby, the reliability of the calculation result of the dioptric power of the eye to be examined wearing an IOL can be improved.

In the ninth aspect of the embodiments, in the eighth aspect, the acquisition step is performed to acquire pupil diameter information representing a pupil diameter, and the calculation step is performed to calculate the dioptric power based on the Hartmann image within a region demarcated based on the pupil diameter information.

According to such a method, the dioptric power is calculated based on the wavefront aberration information corresponding to the pupil diameter. Thereby, the reliability of the calculation result of the dioptric power of the eye to be examined wearing an IOL can be further improved.

In the tenth aspect of the embodiments, in the eighth aspect or the ninth aspect, the intraocular lens information represents either a monofocal type or a multifocal type.

According to such a method, the dioptric power of the eye to be examined wearing the IOL is calculated using a measurement method in accordance with either the monofocal type IOL or the multifocal type IOL. Thereby, the reliability of the calculation result of the dioptric power of the eye to be examined wearing an IOL can be improved.

In the eleventh aspect of the embodiments, in the tenth aspect, when the intraocular lens is determined to be a monofocal type intraocular lens based on the intraocular lens information, the control step is performed to move the focusing lens to a position corresponding to a focal point distance of the intraocular lens and the calculation step is performed to calculate a single dioptric power based on the Hartmann image.

According to such a method, the reliability of the calculation result of the dioptric power of the eye to be examined wearing the monofocal type IOL can be improved.

In the twelfth aspect of the embodiments, in the tenth aspect or the eleventh aspect, when the intraocular lens is determined to be a multifocal refractive type intraocular lens based on the intraocular lens information, the control step is performed to move the focusing lens to a position corresponding to an average focal point distance of the intraocular lens or a position corresponding to a focal point distance of the intraocular lens and the calculation step is performed to calculate the dioptric power for each focal point distance of the intraocular lens.

According to such a method, the reliability of the calculation result of the dioptric power of the eye to be examined wearing the multifocal refractive type IOL can be improved.

In the thirteenth aspect of the embodiments, in any one of the tenth aspect to the twelfth aspect, when the intraocular lens is determined to be a multifocal diffractive type intraocular lens based on the intraocular lens information, the control step is performed to move the focusing lens to a position corresponding to an average focal point distance of the intraocular lens or a position corresponding to a focal point distance of the intraocular lens and the calculation step is performed to calculate the dioptric power based on two or more separated point images that are separated from point images that make up the Hartmann image.

According to such a method, the reliability of the calculation result of the dioptric power of the eye to be examined wearing the multifocal diffractive type IOL can be improved.

In the fourteenth aspect of the embodiments, in any one of the tenth aspect to the thirteenth aspect, when the intraocular lens is determined to be an extended depth of focus type intraocular lens based on the intraocular lens information, the control step is performed to move the focusing lens to a position corresponding to an average focal point distance of the intraocular lens or a position corresponding to a focal point distance of the intraocular lens and the calculation step is performed to calculate the dioptric power based on two focal points of an approximate ellipse identified by performing elliptical approximation on point images that make up the Hartmann image.

According to such a method, the reliability of the calculation result of the dioptric power of the eye to be examined wearing the EDoF type IOL can be improved.

The fifteenth aspect of the embodiments is a program of causing a computer to execute each step of the method of controlling the ophthalmic apparatus of any one of the eighth aspect to the fourteenth aspect.

According to such a program, the dioptric power of the eye to be examined wearing the IOL is calculated using a measurement method in accordance with the type of the IOL worn by the eye to be examined. Thereby, the reliability of the calculation result of the dioptric power of the eye to be examined wearing an IOL can be improved.

### (Other modification examples)

The embodiment described above is merely an example for implementing the present invention. Those who intend to implement the present invention can apply any modification, omission, addition, or the like within the scope of the gist of the present invention.

It is possible to apply the invention according to the above embodiments to apparatuses having arbitrary functions adaptable in the field of ophthalmology. Examples of such functions include a tonometry function, a fundus photography function, an anterior segment photography function, an optical coherence tomography (OCT) function, an ultrasonic examination function, and the like. The intraocular pressure measurement function is realized by the tonometer, etc. The fundus imaging function is realized by the fundus camera, the scanning laser ophthalmoscope (SLO), or the like. The anterior segment imaging function is realized by the slit lamp, etc. The OCT function is realized by the OCT apparatus, etc. The ultrasonic inspection function is realized by the ultrasonic diagnosis apparatus, etc. Further, the present invention can also be applied to an apparatus (multifunctional apparatus) having two or more of such functions.

### [Explanation of symbols]

4 Optotype projection system
5 Observation system
6 Aberration measurement projection system
7 Aberration measurement light reception system
61, 61A, 61B Light source
75 Hartmann plate
76 Area sensor
100 Ophthalmic apparatus
110 Controller
111 Main controller
120 Arithmetic Processor
130 Dioptric power calculator
131 First dioptric power calculator
131A, 132A, 133B, 134A Point image identifying unit
131B, 132B, 133C Representative position identifying unit
131C, 132D, 133E, 134D Zernike polynomial approximate processor
132 Second dioptric power calculator
132C, 133D, 134C Point image group identifying unit
133 Third dioptric power calculator
133A Enhancement processor
134 Fourth dioptric power calculator
134B Elliptical approximate processor
E Eye to be examined
Ef Fundus

## Claims

1. An ophthalmic apparatus, comprising:
a measurement optical system including a focusing lens, and configured to measure wavefront aberration of an eye to be examined wearing an intraocular lens to acquire a Hartmann image;
an acquisition unit configured to acquire intraocular lens information representing at least an optical characteristics of the intraocular lens;
a controller configured to move the focusing lens to a position corresponding to a focal point distance of the intraocular lens determined based on the intraocular lens information and to cause the measurement optical system to acquire the Hartmann image; and
a calculator configured to calculate a dioptric power of the eye to be examined based on the Hartmann image using an arithmetic processing method corresponding to the intraocular lens information.

2. The ophthalmic apparatus of claim 1, wherein
the acquisition unit is configured to acquire pupil diameter information representing a pupil diameter, and
the calculator is configured to calculate the dioptric power based on the Hartmann image within a region demarcated based on the pupil diameter information.

3. The ophthalmic apparatus of claim 1 or 2, wherein
the intraocular lens information represents either a monofocal type or a multifocal type.

4. The ophthalmic apparatus of claim 3, wherein
when the intraocular lens is determined to be a monofocal type intraocular lens based on the intraocular lens information, the controller is configured to move the focusing lens to a position corresponding to a focal point distance of the intraocular lens and the calculator is configured to calculate a single dioptric power based on the Hartmann image.

5. The ophthalmic apparatus of claim 3, wherein
when the intraocular lens is determined to be a multifocal refractive type intraocular lens based on the intraocular lens information, the controller is configured to move the focusing lens to a position corresponding to an average focal point distance of the intraocular lens or a position corresponding to a focal point distance of the intraocular lens and the calculator is configured to calculate the dioptric power for each focal point distance of the intraocular lens.

6. The ophthalmic apparatus of claim 3, wherein
when the intraocular lens is determined to be a multifocal diffractive type intraocular lens based on the intraocular lens information, the controller is configured to move the focusing lens to a position corresponding to an average focal point distance of the intraocular lens or a position corresponding to a focal point distance of the intraocular lens and the calculator is configured to calculate the dioptric power based on two or more separated point images that are separated from point images that make up the Hartmann image.

7. The ophthalmic apparatus of claim 3, wherein
when the intraocular lens is determined to be an extended depth of focus type intraocular lens based on the intraocular lens information, the controller is configured to move the focusing lens to a position corresponding to an average focal point distance of the intraocular lens or a position corresponding to a focal point distance of the intraocular lens and the calculator is configured to calculate the dioptric power based on two focal points of an approximate ellipse identified by performing elliptical approximation on point images that make up the Hartmann image.

8. A method of controlling an ophthalmic apparatus comprising a measurement optical system including a focusing lens and configured to measure wavefront aberration of an eye to be examined wearing an intraocular lens to acquire a Hartmann image, the method comprising:
an acquisition step of acquiring intraocular lens information representing at least an optical characteristics of the intraocular lens;
a control step of moving the focusing lens to a position corresponding to a focal point distance of the intraocular lens determined based on the intraocular lens information and of causing the measurement optical system to acquire the Hartmann image; and
a calculation step of calculating a dioptric power of the eye to be examined based on the Hartmann image using an arithmetic processing method corresponding to the intraocular lens information.

9. The method of controlling the ophthalmic apparatus of claim 8, wherein
the acquisition step is performed to acquire pupil diameter information representing a pupil diameter, and
the calculation step is performed to calculate the dioptric power based on the Hartmann image within a region demarcated based on the pupil diameter information.

10. The method of controlling the ophthalmic apparatus of claim 8 or 9, wherein
the intraocular lens information represents either a monofocal type or a multifocal type.

11. The method of controlling the ophthalmic apparatus of claim 10, wherein
when the intraocular lens is determined to be a monofocal type intraocular lens based on the intraocular lens information, the control step is performed to move the focusing lens to a position corresponding to a focal point distance of the intraocular lens and the calculation step is performed to calculate a single dioptric power based on the Hartmann image.

12. The method of controlling the ophthalmic apparatus of claim 10, wherein
when the intraocular lens is determined to be a multifocal refractive type intraocular lens based on the intraocular lens information, the control step is performed to move the focusing lens to a position corresponding to an average focal point distance of the intraocular lens or a position corresponding to a focal point distance of the intraocular lens and the calculation step is performed to calculate the dioptric power for each focal point distance of the intraocular lens.

13. The method of controlling the ophthalmic apparatus of claim 10, wherein
when the intraocular lens is determined to be a multifocal diffractive type intraocular lens based on the intraocular lens information, the control step is performed to move the focusing lens to a position corresponding to an average focal point distance of the intraocular lens or a position corresponding to a focal point distance of the intraocular lens and the calculation step is performed to calculate the dioptric power based on two or more separated point images that are separated from point images that make up the Hartmann image.

14. The method of controlling the ophthalmic apparatus of claim 10, wherein
when the intraocular lens is determined to be an extended depth of focus type intraocular lens based on the intraocular lens information, the control step is performed to move the focusing lens to a position corresponding to an average focal point distance of the intraocular lens or a position corresponding to a focal point distance of the intraocular lens and the calculation step is performed to calculate the dioptric power based on two focal points of an approximate ellipse identified by performing elliptical approximation on point images that make up the Hartmann image.

15. A program of causing a computer to execute each step of the method of controlling the ophthalmic apparatus of claim 8 or 9.
